# EUROPÄISCHE PATENTANMELDUNG

(11) **EP 4 151 624 A1**
(43) Veröffentlichungstag der Anmeldung: **22.03.2023**
(21) Anmeldenummer: 21198124.6
(22) Anmeldetag: 21.09.2021
(51) Int. Cl.: C07C 291/04, C07D 207/46, C07D 211/94, C11D 1/00

(54) **NEUE AMIN-N-OXID-VERBINDUNGEN**

(71) Anmelder: Karl-Franzens-Universität Graz, 8010 Graz (AT); Rijksuniversiteit Groningen, 9712 CP Groningen (NL)
(72) Erfinder: Barta Weissert, Katalin, 8010 Graz (AT); Hochegger, Markus, 8010 Graz (AT); Fridrich, Bálint, 9747 AG Groningen (NL)
(74) Vertreter: Ellmeyer, Wolfgang

(57) **Zusammenfassung**

Die Erfindung betrifft Amin-N-oxid-Verbindungen der Formel (I) oder (II): 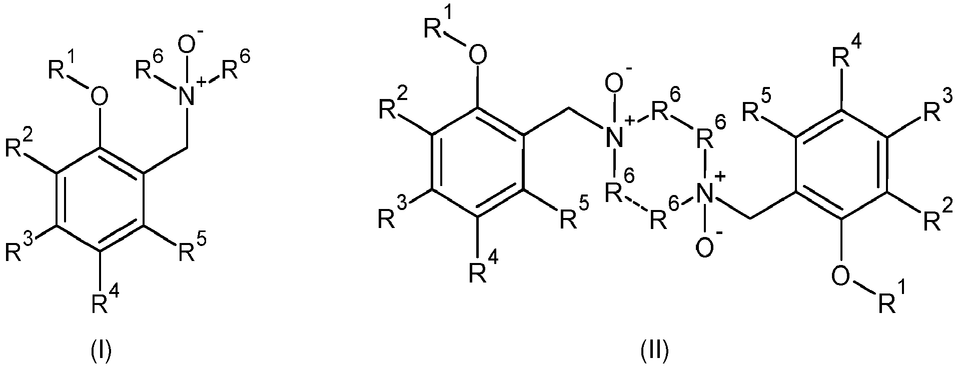 worin
R¹ aus Kohlenwasserstoff-Resten mit 4 bis 26 C-Atomen und gegebenenfalls zumindest einem O- oder S-Atom ausgewählt ist;
R², R³ und R⁵ jeweils unabhängig aus Wasserstoff, R¹-O-, R⁸ und in Formel (I) auch aus -CH₂-N⁺(O⁻)R⁶R⁶ ausgewählt sind, wobei R⁸ für einen Kohlenwasserstoff-Rest mit 1 bis 26 C-Atomen und gegebenenfalls zumindest einem O- oder S-Atom steht;
R⁴ aus Wasserstoff und R⁸ ausgewählt ist; und
die R⁶ jeweils unabhängig aus Kohlenwasserstoff-Resten mit 1 bis 6 C-Atomen und gegebenenfalls zumindest einem N-, O- oder S-Atom ausgewählt sind;
wobei gegebenenfalls zwei an dasselbe Stickstoffatom gebundene Reste R⁶ miteinander zu einem stickstoffhältigen Ring verbunden sind oder
wobei gegebenenfalls einer oder beide Reste R⁶ einer Gruppierung -N⁺(O⁻)R⁶R⁶ mit einem oder beiden Resten R⁶ einer solchen Gruppierung eines anderen Moleküls der Formel (I) verbunden sind und unter Ausbildung einer Brücke mit der Struktur 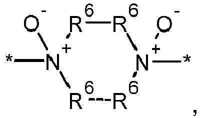 worin die gestrichelte Linie eine optionale Bindung zwischen den beiden R⁶ und die Sternchen die Anbindungen der Brücke an die beiden aromatischen Ringe anzeigen, ein Dimer gemäß Formel (II) bilden;
sowie ein Herstellungsverfahren dafür und ihre Verwendung als Tenside.

## Beschreibung

Die vorliegende Erfindung betrifft neue Amin-N-oxid-Verbindungen, ein Herstellungsverfahren dafür sowie deren Verwendung als Tenside.

### HINTERGRUND DER ERFINDUNG

Lignin stellt ein im Überfluss vorkommendes aromatisches Biopolymer dar, dessen Struktur hauptsächlich auf drei substituierten Phenolen, so genannten Monolignolen (p-Coumaryl-, Coniferyl- und Sinapylalkohol), basiert, die durch eine Vielzahl von unterschiedlichen C-O- und C-C-Bindungen eine amorphe 3-dimensionale Struktur ausbilden. Unterschiedliche Methoden wurden entwickelt, um den katalytischen Abbau von Lignin mittels Depolymerisation zu ermöglichen, um industriell verwertbare monomolekulare Phenol- und/oder Benzaldehyd-Derivate erhalten zu können. Neben Monomeren, können die Produkte dieser Depolymerisationsprozesse auch phenolische Di-, Tri- und Oligomere enthalten.

Erst kürzlich wurden verschiedene Methoden entwickelt, durch welche eine selektive Depolymerisation von Lignin möglich geworden ist, wobei es sich hauptsächlich um reduktive oder oxidative Reaktionsstrategien handelt. Durch Erstere werden üblicherweise Phenol-, Guajacol- oder Syringol-Derivate mit aliphatischen Resten gewonnen, die typischerweise ein bis drei Kohlenstoffatome lang und mit Alkohol-, Aldehyd-, Ester- und/oder Ketonfunktionalitäten versehen sind. Letztere liefern hingegen typischerweise aromatische Aldehyde wie Vanillin oder Syringaldehyd oder ähnlich funktionalisierte Guajacol- und Syringol-Derivate. Die Hauptprodukte solcher Depolymerisationsverfahren umfassen Guajacol und Syringol bzw. Vanillin und Syringaldehyd, die häufig jeweils einen oder mehrere Alkyl- und/oder Alkoxy-Substituenten am aromatischen Ring aufweisen.

All die genannten Ligninabbauprodukte stellen wertvolle Ressourcen auf biologischer Basis dar, aus denen in den letzten Jahren auch eine Reihe verschiedenster Produkte hergestellt wurden. Den Recherchen der Erfinder zufolge befinden sich darunter allerdings kaum Tenside, obwohl auch auf diesem Gebiet ein Bedarf an Produkten besteht, die auf der Grundlage nicht-essbarer nachwachsender Rohstoffe synthetisierbar sind.

Ziel der Erfindung war vor diesem Hintergrund die Synthese neuer chemischer Verbindungen mittels Funktionalisierung dieser Produkte des Ligninabbaus sowie ähnlicher Verbindungen, vorzugsweise auf eine umweltschonende Weise, die sich insbesondere zur Verwendung als Tenside eignen.

### ZUSAMMENFASSUNG DER ERFINDUNG

Dieses Ziel erreicht die vorliegende Erfindung in einem ersten Aspekt durch Bereitstellung von Amin-N-oxid-Verbindungen der nachstehenden Formel (I) oder (II): 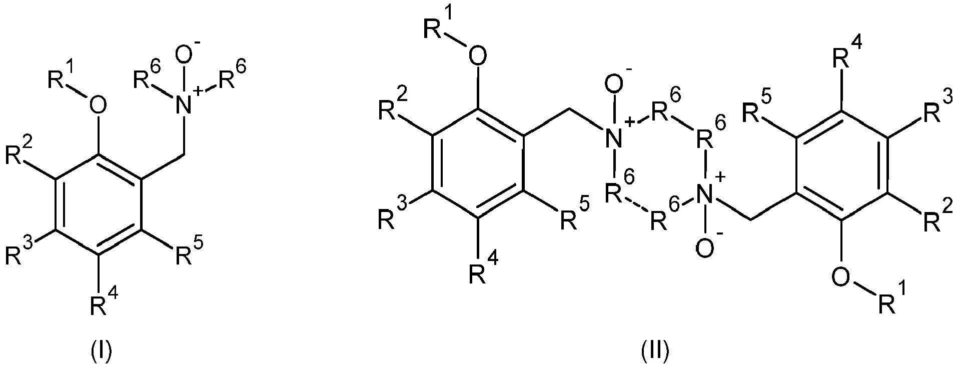 worin
R¹ aus linearen, verzweigten oder zyklischen Kohlenwasserstoff-Resten mit 4 bis 26 Kohlenstoffatomen, in denen gegebenenfalls zumindest ein Kohlenstoffatom durch ein Sauerstoff- oder Schwefelatom ersetzt ist, ausgewählt ist;
R², R³ und R⁵ jeweils unabhängig aus Wasserstoff, R¹-O-, R⁸ und in Formel (I) auch aus -CH₂-N⁺(O⁻)R⁶R⁶ ausgewählt sind, wobei R⁸ für einen linearen, verzweigten oder zyklischen Kohlenwasserstoff-Rest mit 1 bis 26 Kohlenstoffatomen, in dem gegebenenfalls zumindest ein Kohlenstoffatom durch ein Sauerstoff- oder Schwefelatom ersetzt ist, steht;
R⁴ aus Wasserstoff und R⁸ ausgewählt ist; und
die Reste R⁶ jeweils unabhängig aus gesättigten, linearen oder verzweigten Kohlenwasserstoff-Resten mit 1 bis 6 Kohlenstoffatomen, in denen gegebenenfalls zumindest ein Kohlenstoffatom durch ein Stickstoff-, Sauerstoff- oder Schwefelatom ersetzt ist, ausgewählt sind;
wobei gegebenenfalls zwei an dasselbe Stickstoffatom gebundene Reste R⁶ miteinander zu einem fünf- oder sechsgliedrigen stickstoffhältigen Ring verbunden sind oder
wobei gegebenenfalls ein Rest R⁶ oder beide Reste R⁶ einer Amin-N-oxid-Gruppierung -N⁺(O⁻)R⁶R⁶ mit einem oder beiden Resten R⁶ einer solchen Gruppierung eines anderen Moleküls der Formel (I) verbunden sind und unter Ausbildung einer Brücke mit der Struktur 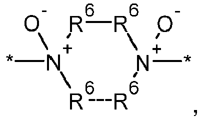 worin die gestrichelte Linie eine optionale Bindung zwischen den beiden Resten R⁶ und die Sternchen die Anbindungen der Brücke an die beiden aromatischen Ringe anzeigen, ein Dimer gemäß Formel (II) bilden.

Derartige Amin-N-oxid-Monomere der Formel (I) oder entsprechende Dimere der Formel (II) lassen sich, wie die Erfinder festgestellt haben, auf relativ einfache sowie umweltschonende Weise aus leicht verfügbaren Lignin-Abbauprodukten herstellen und eignen sich ausgezeichnet als Tenside. Aufgrund der starken Hydrophilie der N-Oxid-Gruppierung(en) und der Hydrophobie des bzw. der Aromaten reicht sogar eine einstellige Anzahl an Kohlenstoffatomen in den Resten R¹ bis R⁵ aus, um den Verbindungen die erforderliche Amphiphilie zu verleihen.

Vorzugsweise beträgt die Anzahl an Kohlenstoffatomen in den Resten R¹ bis R⁵ jedoch zumindest 9 Kohlenstoffatome. Dies ist vor allem im Hinblick auf die Hydrophobie zu bevorzugen, wenn zwei oder mehr Amin-N-Oxid-Gruppierungen -CH₂-N⁺(O⁻)R⁶R⁶ an den Aromaten gebunden sind. Aber auch der Umstand, dass zu den Hauptprodukten der Depolymerisation von Lignin neben den eingangs erwähnten Derivaten von Guajacol und Syringol auch solche von Brenzcatechin zählen, insbesondere jeweils ein- oder zweifach mit Niederalkyl und/oder -alkoxy substituierte Derivate, vereinfacht die Synthese von Amin-N-oxid-Verbindungen mit zumindest 9 Kohlenstoffatomen, da die jeweiligen freien phenolischen OH-Gruppen bloß mit leicht verfügbaren und biologisch abbaubaren Fettalkylresten verethert zu werden brauchen.

Da einerseits Fettalkohole in der Natur sowohl in gesättigter als auch in ungesättigter Form, d.h. mit einer oder mehreren C=C-Doppelbindungen vorkommen, und andererseits, wie eingangs erwähnt, die Lignin-Abbauprodukte auch mehr als einen aromatischen, aber auch nicht-aromatische Ringe (z.B. Dioxolan) als Substituenten umfassen können, sind in der Definition von R¹ bis R⁵ gemäß vorliegender Erfindung sowohl gesättigte als auch ungesättigte sowie zyklische Reste umfasst.

Dass neben den Amin-N-oxid-Monomeren der Formel (I) auch Dimere der Formel (II) von der vorliegenden Erfindung umfasst sind, liegt an der mit einer Aminoalkylierungsreaktion nach Betti/Mannich beginnenden Synthese, die mit sekundären Mono- und Diaminen auf analoge Weise abläuft, was in Bezug auf den zweiten Aspekt der Erfindung näher erläutert und durch die späteren Beispiele belegt wird.

Die untere und obere Grenze für die Anzahl der Kohlenstoffatome in den Resten R¹ bis R⁵ bezieht sich auf die bevorzugte Verwendung von Fettalkylresten zur Veretherung von freien phenolischen OH-Gruppen in den Ausgangsprodukten, für deren Kettenlänge in der Literatur zwischen 4 bis 6 als Untergrenze und zwischen 22 und 26 als Obergrenze angegeben werden. Erfindungsgemäß bevorzugt wird eine maximale Länge von 18 Kohlenstoffatomen für die im Syntheseverfahren mittels Veretherung eingeführten Fettalkylreste bzw. von 4 Kohlenstoffatomen für die bereits im Ausgangsmaterial an den Aromaten gebundenen Alkyl- bzw. Alkoxy- oder gegebenenfalls auch Alkylthio-Resten, was insbesondere für den Rest R⁴ in p-Stellung zur Ethergruppe R¹-O- gilt.

Die Option, dass manche Kohlenstoffatome durch Sauerstoff oder Schwefel ersetzt sein können, bezieht sich ebenfalls vor allem auf das Substitutionsmuster der vorzugsweise durch Lignin-Depolymerisation erhaltenen Ausgangsverbindungen, die, wie eingangs erwähnt, verschiedene Sauerstoff-hältige Funktionalitäten, mitunter aber auch Schwefel-Analoga davon aufweisen können. Andere Heteroatome, wie z.B. Halogene oder Stickstoff, kommen in solchen Verbindungen hingegen kaum vor. Während erstere das Syntheseverfahren gemäß dem zweiten Aspekt der Erfindung nicht stören würden, würden jedoch Stickstoffatome in den Resten R¹ bis R⁵ im abschließenden Oxidationsschritt mit hoher Wahrscheinlichkeit ebenfalls zu N-Oxiden oxidiert werden, was die Hydrophobie dieses Abschnitts der erfindungsgemäßen Verbindungen senken würde, vor allem, wenn die Stickstoffatome nicht sehr nahe zum aromatischen Ring positioniert wären. Für die Zwecke der vorliegenden Erfindung brauchen daher andere Heteroatome als Sauerstoff und Schwefel nicht in Betracht gezogen zu werden.

Die Option, dass einer oder mehrere der Reste R², R³ und R⁵ auch eine Amin-N-oxid-Gruppierung -CH₂-N⁺(O⁻)R⁶R⁶ darstellen können, bezieht sich hingegen gezielt auf das Syntheseverfahren, in dessen erstem Schritt der aromatische Ring auch an mehr als einer Position aminoalkyliert werden kann - und auch wurde, wie die Beispiele belegen.

In manchen bevorzugten Ausführungsformen der Erfindung ist daher R¹ C₆-C₂₂-Alkyl, noch bevorzugter C₈-C₁₈-Alkyl.

Alternativ oder zusätzlich dazu ist in manchen bevorzugten Ausführungsformen R² aus C₁-C₂₂-Alkyl, C₁-C₂₂-Alkoxy und -CH₂-N⁺(O⁻)R⁶R⁶, noch bevorzugter aus C₁-C₁₈-Alkoxy und -CH₂-N⁺(O⁻)R⁶R⁶, ausgewählt. Alternativ oder zusätzlich dazu sind in bevorzugten Ausführungsformen R³ und R⁵ aus Wasserstoff und -CH₂-N⁺(O⁻)R⁶R⁶ ausgewählt, wobei besonders bevorzugt einer von R³ und R⁵ Wasserstoff ist und der andere -CH₂-N⁺(O⁻)R⁶R⁶ ist. Dabei bezieht sich die Option -CH₂-N⁺(O⁻)R⁶R⁶ für R², R³ bzw. R⁵, wie zuvor erwähnt, auf eine mehrfache Aminoalkylierung des Aromaten.

Alternativ oder zusätzlich dazu ist in manchen bevorzugten Ausführungsformen R⁴ aus Wasserstoff, C₁-C₄-Alkyl und C₁-C₄-Alkoxy und noch bevorzugter aus Wasserstoff und C₁-C₄-Alkyl ausgewählt und ist noch bevorzugter C₁-C₄-Alkyl.

In manchen besonders bevorzugten Ausführungsformen ― nach nur einfacher Aminoalkylierung im Syntheseverfahren ― gilt Folgendes:
R¹ ist C₈-C₁₈-Alkyl,
R² ist C₁-C₁₈-Alkoxy;
R³ und R⁵ sind jeweils Wasserstoff; und
R⁴ ist C₁-C₄-Alkyl, insbesondere Ethyl oder Propyl.

Falls als Ausgangssubstanz ein Derivat von Brenzcatechin mit zwei vicinalen phenolischen OH-Gruppen im Syntheseverfahren eingesetzt wurde, ist R² besonders bevorzugt C₁-C₁₈-Alkoxy, ansonsten, z.B. bei Verwendung eines Derivats von Guajacol oder Syringol, ist R² jedoch insbesondere Methoxy. In letzterem Fall, bei Verwendung von Syringol, ist gleichzeitig auch R⁵ Methoxy.

In manchen besonders bevorzugten Ausführungsformen ― nach doppelter Aminoalkylierung im Syntheseverfahren ― gilt hingegen Folgendes:
R¹ ist C₈-C₁₈-Alkyl,
R² ist -CH₂-N⁺(O⁻)R⁶R⁶;
R³ und R⁵ sind jeweils Wasserstoff; und
R⁴ ist C₁-C₄-Alkyl, insbesondere Ethyl oder Propyl.

In Bezug auf die Reste R⁶, die allgemein bis zu sechs Kohlenstoff- und gegebenenfalls Heteroatome (O, S oder speziell N) umfassen können, gilt in manchen bevorzugten Ausführungsformen des ersten Aspekts der vorliegenden Erfindung, dass sie jeweils unabhängig aus Methyl, Ethyl und Dimethylaminoethyl ausgewählt sind.

Alternativ oder zusätzlich dazu gilt in manchen bevorzugten Ausführungsformen, dass zwei an dasselbe Stickstoffatom gebundene Reste R⁶ miteinander verbunden sind und zusammen mit dem Stickstoffatom eine der nachstehenden Gruppen bilden: 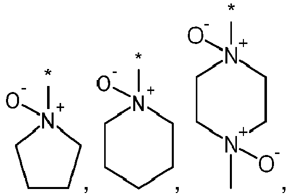 wobei das Sternchen jeweils die Anbindung an den aromatischen Ring anzeigt.

In manchen besonders bevorzugten Ausführungsformen sind die Reste R⁶ jeweils Methyl, und eine oder beide Methylgruppen einer Gruppierung -N⁺(O⁻)(CH₃)₂ ist/sind mit einer oder beiden Methylgruppen einer solchen Gruppierung eines anderen Moleküls der Formel (I) verbunden, so dass diese unter Ausbildung einer Brücke mit der Struktur 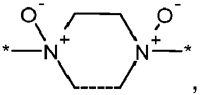 worin die gestrichelte Linie eine optionale Bindung zwischen den beiden Methylgruppen und die Sternchen die Anbindungen der Brücke an die beiden aromatischen Ringe anzeigen, ein Dimer der Amin-N-oxid-Verbindung gemäß Formel (II) bilden.

Insbesondere ist die Amin-N-oxid-Verbindung gemäß dem ersten Aspekt der vorliegenden Erfindung aus den folgenden Verbindungen ausgewählt:
N,N-Dimethyl-1-(5-ethyl-3-methoxy-2-octyloxyphenyl)methanamin-N-oxid (1) 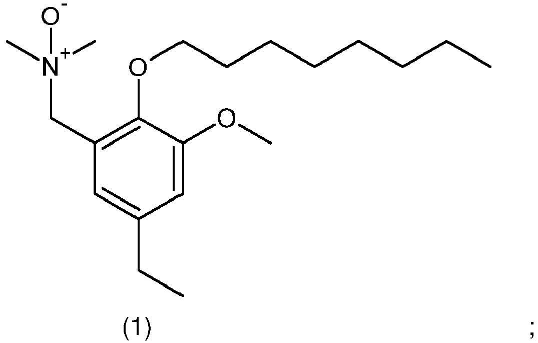
N,N-Dimethyl-1-(2-decyloxy-5-ethyl-3-methoxyphenyl)methanamin-N-oxid (2) 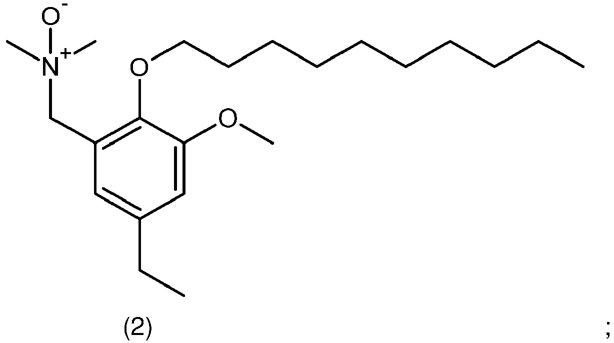
N,N-Dimethyl-1-(2-dodecyloxy-5-ethyl-3-methoxyphenyl)methanamin-N-oxid (3) 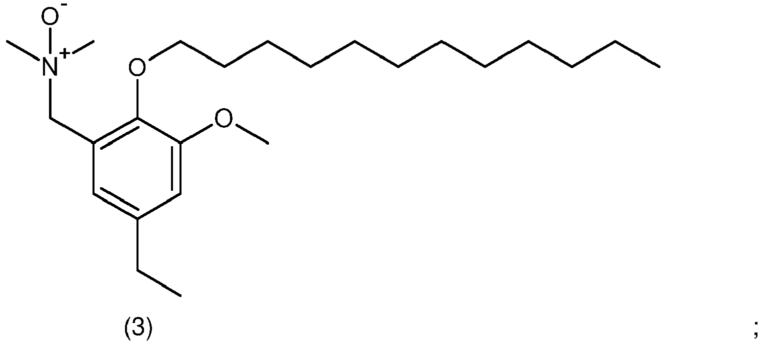
N,N-Dimethyl-1-(5-ethyl-3-methoxy-2-tetradecyloxyphenyl)methanamin-N-oxid (4) 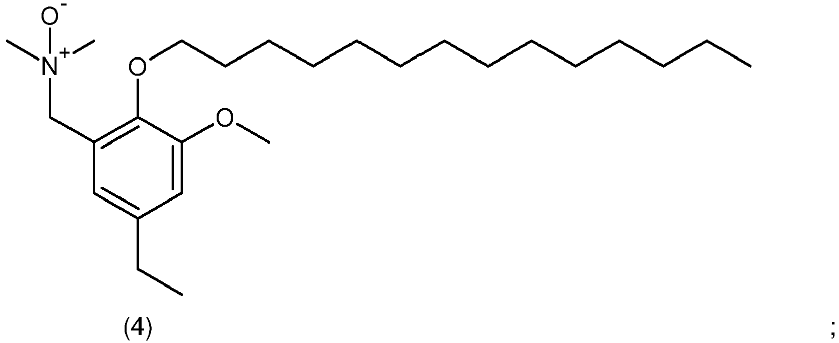
N,N-Dimethyl-1-(5-ethyl-2-hexadecyloxy-3-methoxyphenyl)methanamin-N-oxid (5) 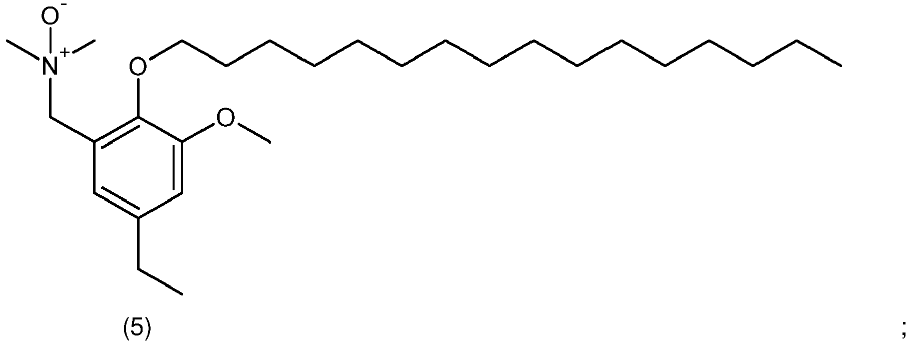
N,N-Dimethyl-1-(5-ethyl-3-methoxy-2-octadecyloxyphenyl)methanamin-N-oxid (6) 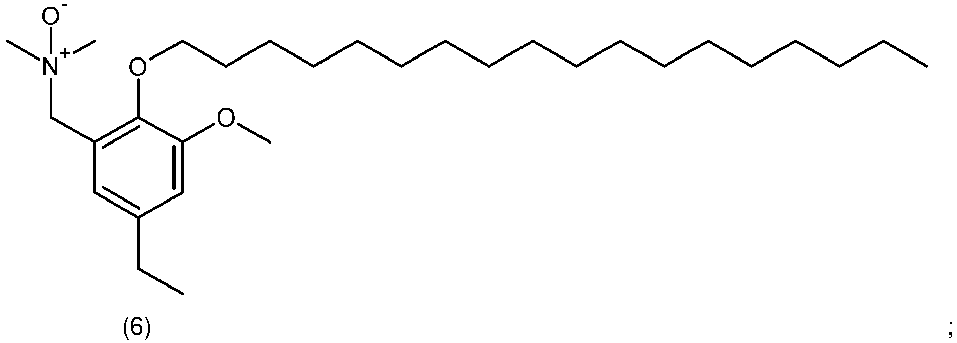
1-(2-Dodecyloxy-5-ethyl-3-methoxybenzyl)pyrrolidin-1-oxid (7) 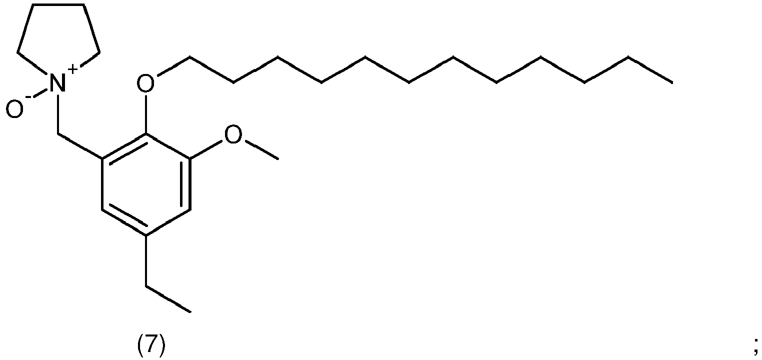
1-(2-Dodecyloxy-5-ethyl-3-methoxybenzyl)piperidin-1-oxid (8) 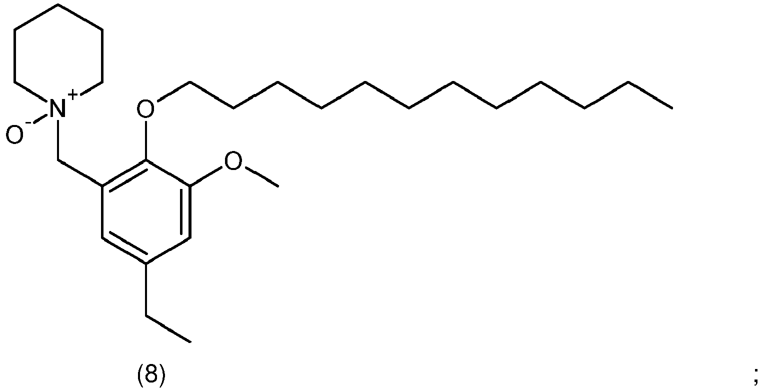
1-(2-Dodecyloxy-5-ethyl-3-methoxybenzyl)-4-methylpiperazin-1,4-dioxid (9) 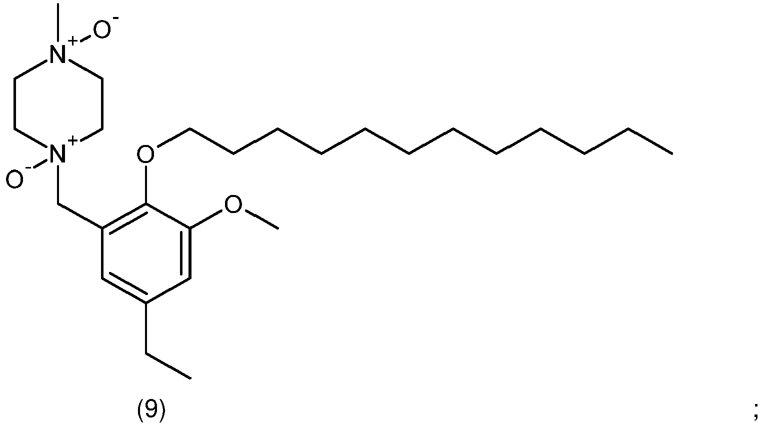
N,N-Dimethyl-N'-(2-dodecyloxy-5-ethyl-3-methoxybenzyl)-N'-methylethan-1,2-diamin-di-N-oxid (10) 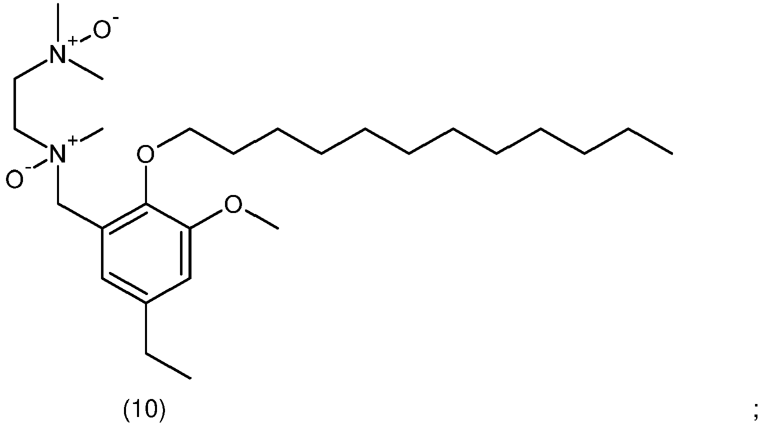
1,1'-(2,3-Dioctyloxy-5-ethyl-1,4-phenylen)-bis(N,N-dimethylmethanamin-N-oxid) und 1,1'-(2,3-Dioctyloxy-5-ethyl-1,6-phenylen)-bis(N,N-dimethylmethanamin-N-oxid) (11) 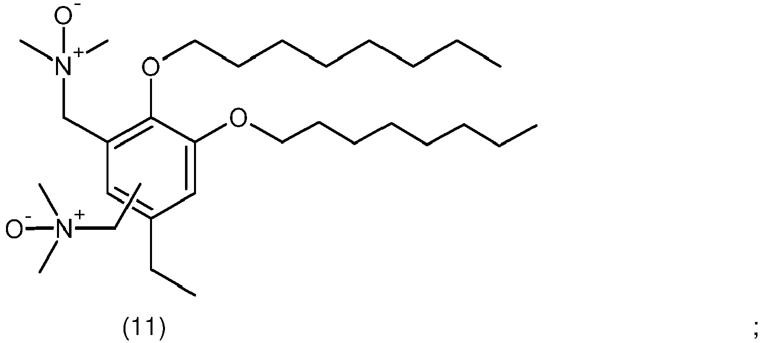
1,1'-(5-Ethyl-2-octyloxy-1,3-phenylen)-bis(N,N-dimethylmethanamin-N-oxid) (12) 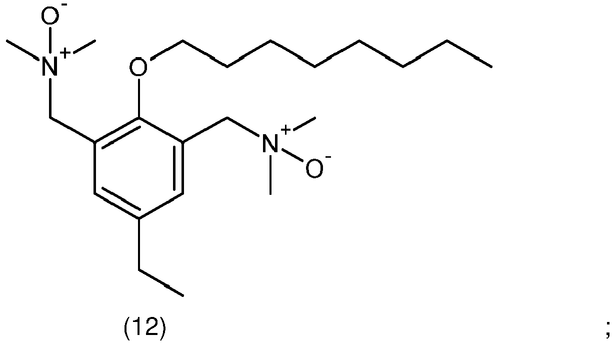
1,1'-(2-Dodecyloxy-5-ethyl-1,3-phenylen)-bis(N,N-dimethylmethanamin-N-oxid) (13) 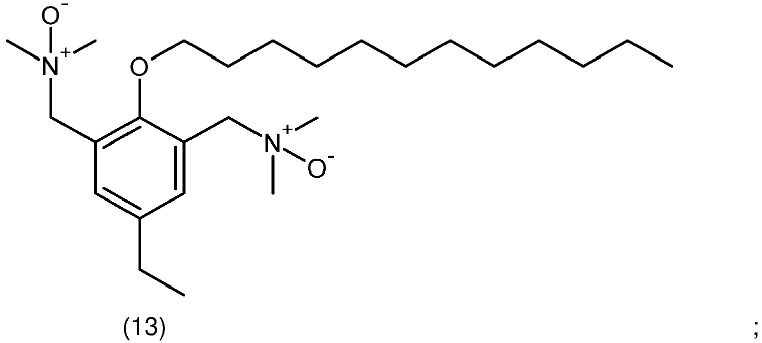
1,1'-(5-Ethyl-2-hexadecyloxy-1,3-phenylen)-bis(N,N-dimethylmethanamin-N-oxid) (14) 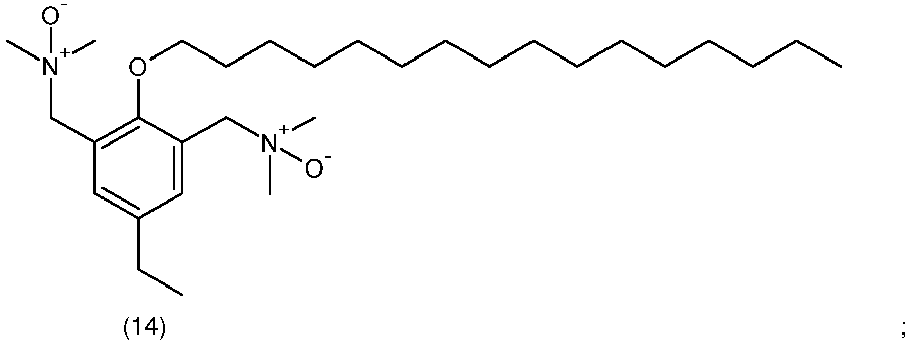
1,1'-(5-Ethyl-2-octadecyloxy-1,3-phenylen)-bis(N,N-dimethylmethanamin-N-oxid) (15) 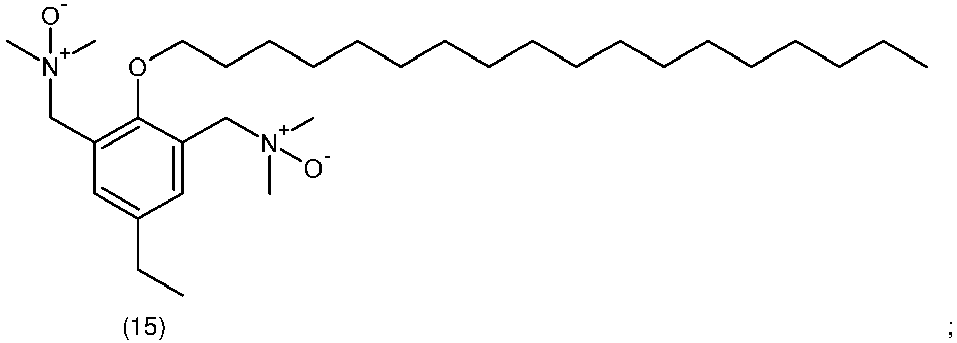
1,4-Bis(5-ethyl-3-methoxy-2-octyloxybenzyl)piperazin-1,4-dioxid (16) 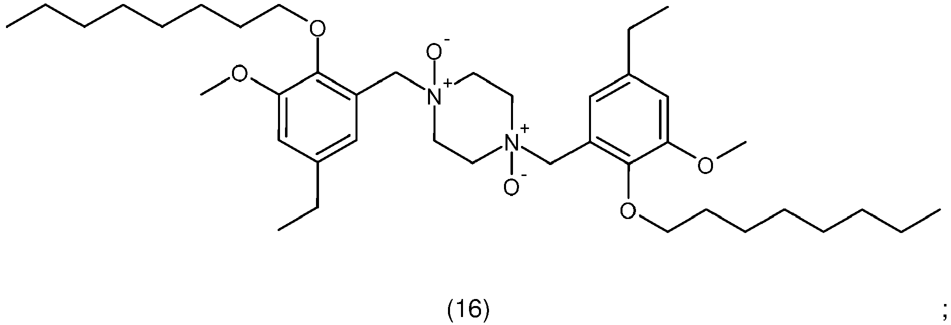
1,4-Bis(2-dodecyloxy-5-ethyl-3-methoxybenzyl)piperazin-1,4-dioxid (17) 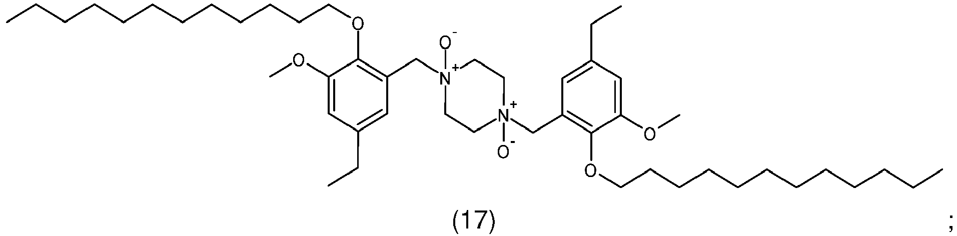
N,N'-Bis(5-ethyl-3-methoxy-2-octyloxybenzyl)-N,N'-dimethylethan-1,2-diamin-di-N-oxid (18)

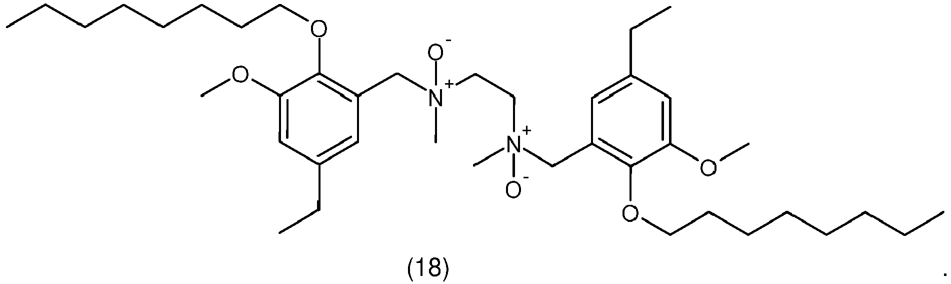

In einem zweiten Aspekt betrifft die vorliegende Erfindung ein Verfahren zur Herstellung einer Amin-N-oxid-Verbindung gemäß dem ersten Aspekt, das die folgenden Schritte umfasst:
1) das Umsetzen eines Phenol-Derivats der nachstehenden Formel (III): 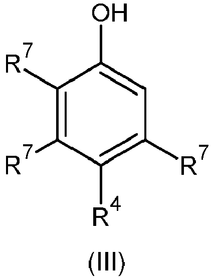 worin
   R⁴ aus Wasserstoff und R⁸ ausgewählt ist, wobei R⁸ für einen linearen, verzweigten oder zyklischen Kohlenwasserstoff-Rest mit 1 bis 26 Kohlenstoffatomen, in dem gegebenenfalls zumindest ein Kohlenstoffatom durch ein Sauerstoff- oder Schwefelatom ersetzt ist, steht, und
   die R⁷ jeweils unabhängig aus Wasserstoff, Hydroxy und R⁸ ausgewählt sind, mit einem sekundären Amin HNR⁶R⁶, worin die Reste R⁶ jeweils unabhängig aus gesättigten, linearen oder verzweigten Kohlenwasserstoff-Resten mit 1 bis 6 Kohlenstoffatomen, in denen gegebenenfalls zumindest ein Kohlenstoffatom durch ein Stickstoff-, Sauerstoff- oder Schwefelatom ersetzt ist, ausgewählt sind,
   wobei gegebenenfalls die beiden Reste R⁶ miteinander zu einem fünf- oder sechsgliedrigen stickstoffhältigen Ring verbunden sind oder
   wobei gegebenenfalls jeweils ein Rest R⁶ oder jeweils beide Reste R⁶ zweier Moleküle des sekundären Amins unter Ausbildung eines Dimers des sekundären Amins mit der Struktur 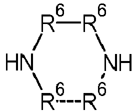 miteinander verbunden sind, worin die gestrichelte Linie eine optionale Bindung zwischen den beiden Resten R⁶ anzeigt,
      mittels einer Aminoalkylierungsreaktion nach Betti/Mannich in Gegenwart von Formaldehyd in einem polaren Lösungsmittel, wodurch das Wasserstoffatom in ortho-Stellung zur phenolischen OH-Gruppe und gegebenenfalls ein weiteres substituierbares Wasserstoffatom R⁷ des Phenol-Derivats der Formel (II) durch eine Gruppierung -CH₂-NR⁶R⁶ ersetzt wird/werden und eine entsprechende Betti-Base der Formel (IV) oder (V) erhalten wird: 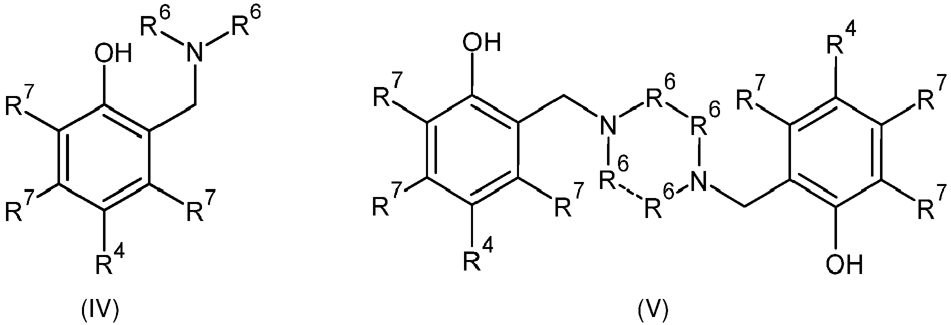 worin die R⁷ jeweils unabhängig aus Wasserstoff, Hydroxy, R⁸ und in Formel (IV) auch aus -CH₂-NR⁶R⁶ ausgewählt sind und die gestrichelte Linie eine optionale Bindung zwischen den beiden Resten R⁶ anzeigt;
2) das Umsetzen der (beiden) phenolischen OH-Gruppe(n) und gegebenenfalls weiterer freier OH-Gruppen R⁷ der jeweiligen Betti-Base der Formel (IV) oder (V) mit einer Verbindung der Formel R¹-X, worin R¹ aus linearen, verzweigten oder zyklischen Kohlenwasserstoff-Resten mit 4 bis 26 Kohlenstoffatomen, in denen gegebenenfalls zumindest ein Kohlenstoffatom durch ein Sauerstoff- oder Schwefelatom ersetzt ist, ausgewählt ist und X für eine aus Halogeniden und Sulfonaten ausgewählte Abgangsgruppe steht, mittels einer Veretherungsreaktion nach Williamson in Gegenwart einer Base in einem organischen Lösungsmittel oder ohne Lösungsmittel, wodurch ein entsprechender Ether der Formel (VI) oder (VII) erhalten wird: 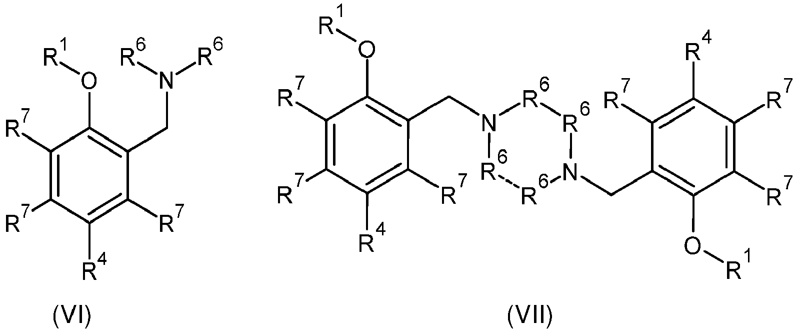 worin die R⁷ jeweils unabhängig aus Wasserstoff, R¹-O-, R⁸ und in Formel (VI) auch aus -CH₂-NR⁶R⁶ ausgewählt sind und die gestrichelte Linie eine optionale Bindung zwischen den beiden Resten R⁶ anzeigt; und
3) das Oxidieren jeglicher Aminogruppen -NR⁶R⁶ des jeweiligen Ethers der Formel (VI) oder (VII) durch Umsetzung mit einem Oxidationsmittel in Wasser, einem organischen Lösungsmittel oder einem Gemisch davon, wodurch die Amin-N-oxid-Verbindung der Formel (I) oder (II) erhalten wird: 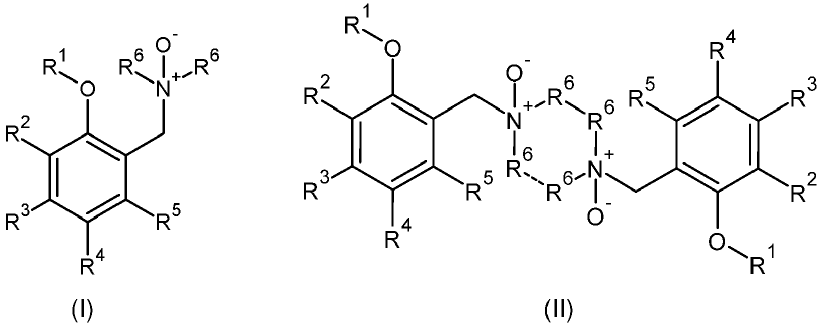
worin R², R³ und R⁵ jeweils unabhängig aus Wasserstoff, R¹-O-, R⁸ und in Formel (I) auch aus -CH₂-N⁺(O⁻)R⁶R⁶ ausgewählt sind und die gestrichelte Linie eine optionale Bindung zwischen den beiden Resten R⁶ anzeigt.

Auf diese Weise ist es gemäß vorliegender Erfindung möglich, durch ein vergleichsweise einfaches und kostengünstiges Verfahren, das eine Abfolge an sich bekannter Einzelreaktionen umfasst, aus Phenol-Derivaten der Formel (III) Amin-N-oxid-Verbindungen zu synthetisieren. In bevorzugten Ausführungsformen handelt es sich bei den Ausgangsverbindungen um leicht verfügbare Produkte der Lignin-Depolymerisation, und das Verfahren wird auf möglichst umweltschonende Weise durchgeführt.

Dazu zählt unter anderem, dass in bevorzugten Ausführungsformen des Verfahrens die Aminoalkylierung nach Betti/Mannich in Schritt 1) in Wasser und noch bevorzugter bei Raumtemperatur durchgeführt wird, wodurch der Einsatz von Lösungsmitteln und großen Energiemengen vermieden wird und überraschenderweise auch die Ausbeuten gesteigert werden können. Allerdings können organische Lösungsmittel, wie z.B. Alkohole wie Methanol, Ethanol oder (Iso-)Propanol, oder Acetonitril oder Toluol, dennoch eingesetzt werden, und das jeweils anstelle oder auch im Gemisch mit Wasser. Bevorzugt wird jedoch die Verwendung von Wasser als alleiniges Lösungsmittel, sofern die Löslichkeit des Phenol-Derivats der Formel (III) dies zulässt.

Letzteres wird in Schritt 1) vorzugsweise mit jeweils 1,05 und noch bevorzugter 1,5 Äquivalenten des sekundären Amins und von Formaldehyd umgesetzt, um eine vollständige Aminoalkylierung an nur einer Position des Aromaten zu gewährleisten, falls eine monomere Amin-N-oxid-Verbindung der Formel (I) gewünscht wird. Soll eine doppelte Aminoalkylierung erfolgen, wird als Ausgangsverbindung vorzugsweise ein an den beiden o-Positionen unsubstituiertes, in p-Stellung aber sehr wohl substituiertes, z.B. alkyliertes, Phenol-Derivat der Formel (III) eingesetzt, um die beiden einzuführenden -CH₂-NR⁶R⁶ Gruppierungen jeweils an eine o-Position zu dirigieren. Ist nur ein o-Wasserstoffatom substituierbar, werden häufig Isomerengemische der doppelt aminoalkylierten Betti-Base der Formel (IV) erhalten. Soll jedoch eine dimere Amin-N-oxid-Verbindung der Formel (II) hergestellt werden, so wird vorzugsweise das Phenol-Derivat der Formel (III) gegenüber dem sekundären Diamin (oder Dimer des sekundären Amins) in einem Verhältnis von 2:1 eingesetzt.

Alternativ oder zusätzlich dazu wird in manchen bevorzugten Ausführungsformen des Verfahrens im Veretherungsschritt 2) jeweils eine Fest-Flüssig-Phasentransferreaktion unter Verwendung einer festen Base und in Gegenwart eines Phasentransfer-Katalysators durchgeführt, um die Umsätze zu erhöhen. Versuche der Erfinder unter Einsatz von Standard-Arbeitsvorschriften der Veretherung nach Williamson, d.h. einphasig in verschiedenen Lösungsmitteln unter Einsatz einer Lösung verschiedener Basen und bei verschiedenen Temperaturen, haben zwar ebenfalls jeweils zur gewünschten Verbindung geführt, allerdings kam es dabei in unterschiedlichem Ausmaß zur Bildung von Nebenprodukten. Der Grund dafür ist, dass Betti-Basen der Formel (IV) mit einer freien OH-Gruppe in ortho-Stellung zur Zersetzung unter Bildung des jeweiligen o-Chinonmethids, wie nachstehend gezeigt, neigen: 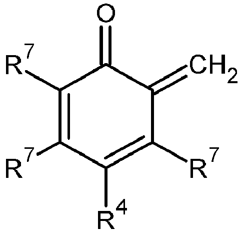 o-Chinonmethide sind ihrerseits sehr reaktiv und neigen zur Polymerisation, weswegen Aminoalkylierungs-Reaktionen nach Betti unter Verwendung ähnlicher Ausgangsverbindungen in der Literatur völlig unbekannt sind.

Der Umstand, dass sowohl Wärme als auch die Gegenwart von Basen (oder Säuren) die Zersetzung der Betti-Basen und die Polymerisation der o-Chinonmethide fördern, stellt einerseits einen weiteren Grund für die bevorzugte Reaktionsführung in Schritt 1) und andererseits jenen für die Entwicklung der Fest-Flüssig-Phasentransferreaktion in Schritt 2) durch die vorliegenden Erfinder dar. Dies trifft insbesondere für die dimeren Betti-Basen der Formel (V) zu, die aufgrund der Gegenwart zweier anfälliger Gruppierungen noch eher zu Zersetzung und Polymerisation neigen.

In besonders bevorzugten Ausführungsformen wird die Base in fester Form zur jeweiligen Betti-Base der Formel (IV) oder (V) zugesetzt und die Reaktanten entweder in einem organischen Lösungsmittel oder auch ohne Lösungsmittel, insbesondere bei Raumtemperatur, miteinander umgesetzt. Besonders bevorzugt werden dabei als Abgangsgruppe X das Chlorid oder Bromid, insbesondere das Bromid, sowie ein wasserfreies Lösungsmittel eingesetzt, um die Bildung von Nebenprodukten zu unterdrücken. Speziell wasserfreies 2-Methyltetrahydrofuran hat sich, nach einer Reihe von Versuchen mit anderen Lösungsmitteln, wie z.B. Acetonitril, bewährt, da es die Umsätze und die Selektivitäten für das gewünschte Produkt am stärksten zu fördern imstande war. Als Abgangsgruppe kann auch ein Sulfonat, wie z.B. Mesylat oder Tosylat, eingesetzt werden, allerdings wäre der Einsatz langkettiger Fettalkoholsulfonate unökonomisch, da diese ja selbst bereits Tenside sind.

Als Base wurden, wie erwähnt, zunächst verschiedene wässrige Lösungen von Alkalimetallcarbonaten und -hydroxiden untersucht, wonach aus den genannten Gründen zu festen Pulvern der Base übergegangen wurde, wobei sich NaOH und besonders gepulverte KOH bewährt haben. Als Phasentransfer-Katalysator wird gemäß vorliegender Erfindung besonders bevorzugt Tetra-n-butylammoniumbromid (TBAB) eingesetzt, obwohl auch andere gängige Katalysatoren, wie z.B. eine Vielzahl anderer quaternärer Ammonium-Verbindungen, eingesetzt werden können.

In bevorzugten Ausführungsformen des erfindungsgemäßen Verfahrens wird auch Schritt 3) auf schonende, umweltfreundliche Weise und mit möglichst quantitativem Umsatz durchgeführt, da sich die Reinigung von amphiphilen Molekülen üblicherweise recht aufwändig gestaltet. In manchen dieser bevorzugten Ausführungsformen wird eine wässrige Lösung von H₂O₂ als Oxidationsmittel eingesetzt, wobei gegebenenfalls Ameisensäuremethylester als zusätzliches Lösungsmittel zugesetzt wird, wobei der Ether der Formel (VI) oder (VII) noch bevorzugter mit 2,5 bis 3 Äquivalenten H₂O₂ umgesetzt wird, um vollständigen Umsatz zu gewährleisten.

Zwar sind auch organische Lösungsmittel wie Dichlormethan oder Acetonitril, mitunter unter Einsatz von Katalysatoren, geeignet, allerdings hat sich eine (z.B. 30%ige) wässrige Lösung von H₂O₂ als Oxidationsmittel bestens bewährt. Zur Erhöhung der Löslichkeit, speziell im Falle der dimeren Amin-N-oxide, können geringe Mengen an organischem Lösungsmittel zugesetzt werden, zu welchem Zweck erfindungsgemäß Ameisensäuremethylester bevorzugt wird.

Und in einem dritten Aspekt betrifft die vorliegende Erfindung die Verwendung der neuen Amin-N-oxid-Verbindungen der Formel (I) oder (II), in denen die Gesamtanzahl der Kohlenstoffatome der Reste R¹ bis R⁵ zumindest 9 betragen sollte, als Tenside.

### BEISPIELE

Die vorliegende Erfindung wird nachstehend anhand von Beispielen näher beschrieben, die jedoch nicht als Einschränkung des Schutzumfangs zu verstehen sind. Zur Illustration wurden zwei repräsentative Modellverbindungen für die als Ausgangssubstanzen im erfindungsgemäßen Verfahren bevorzugten häufigen Lignin-Depolymerisationsprodukte eingesetzt. Zu diesem Zweck wurde je ein Phenol- und ein Diphenol-Derivat, nämlich 4-Ethylguajacol (4-Ethyl-2-methoxyphenol) und 4-Ethylbrenzcatechin (1,2-Dihydroxy-4-ethylbenzol) zu den erfindungsgemäßen Amin-N-oxid-Verbindungen umgesetzt:

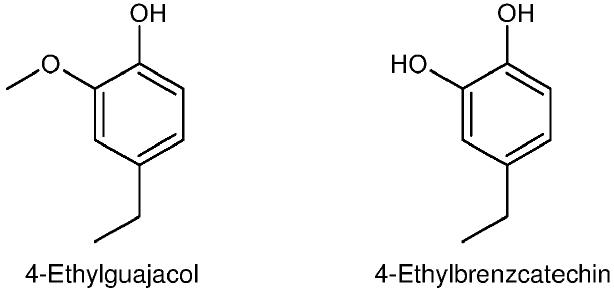

Diese wurden zunächst mit verschiedenen sekundären Aminen und Diaminen in Gegenwart von Formaldehyd einfach oder doppelt aminomethyliert, um entsprechende Betti-Basen zu erhalten, die anschließend mit einer Reihe von Fettalkylhalogeniden einfach oder doppelt verethert und zuletzt zu den Amin-N-oxiden oxidiert wurden.

### Beispiel 1

Herstellung von N,N-Dimethyl-1-(5-ethyl-3-methoxy-2-octyloxyphenyl)methanamin-N-oxid (1)

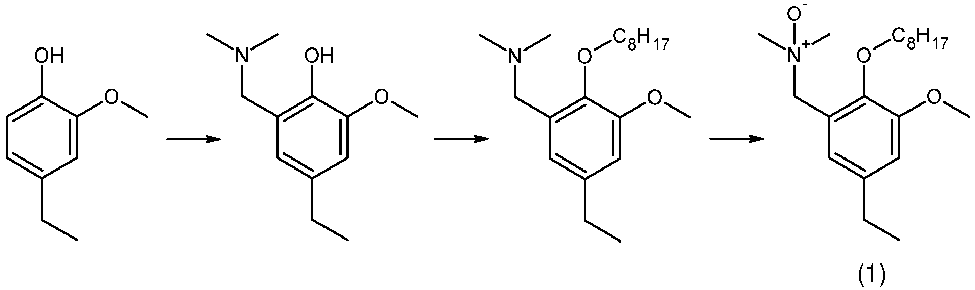

### Stufe 1:

### Variante 1.1:

Eine wässrige Lösung von 4-Ethylguajacol (15,20 g, 100 mmol) wurde binnen 15 min unter konstantem Rühren zu einer 40-gew.-%igen wässrigen Lösung von Dimethylamin (6,76 g, 150 mmol) in einem Eiswasserbad zugetropft. Dazu wurde Paraformaldehyd (4,50 g, 150 mmol) in Aliquoten von jeweils 0,5 g alle 10 min zugesetzt und 3 h lang im Eiswasserbad und danach 9 h lang bei Raumtemperatur gerührt. Anschließend wurden am Rotationsverdampfer die flüchtigen Bestandteile bei Raumtemperatur und danach das Wasser bei 50 °C im Vakuum entfernt, wonach der Rückstand im Vakuumexsikkator vollständig getrocknet wurde. Das aminomethylierte Zwischenprodukt, 2-Dimethylaminomethyl-4-ethyl-6-methoxyphenol, wurde als viskoses, gelbes Öl erhalten (Ausbeute: 20,83 g; 99,5 % d.Th.).

### Variante 1.2:

Eine wässrige Lösung von 4-Ethylguajacol (15,20 g, 100 mmol) wurde binnen 15 min unter konstantem Rühren zu einer 40-gew.-%igen wässrigen Lösung von Dimethylamin (4,56 g, 101 mmol) in einem Eiswasserbad zugetropft. Dazu wurde Paraformaldehyd (4,50 g, 150 mmol) in Aliquoten von jeweils 0,5 g alle 10 min zugesetzt und 3 h lang im Eiswasserbad und danach 72 h lang bei Raumtemperatur gerührt. Anschließend wurde das Reaktionsgemisch 5x mit 25 ml Petrolether (Kp.: 40-60 °C) extrahiert, und die vereinigten organischen Phasen wurden am Rotationsverdampfer im Vakuum eingeengt, wonach der Rückstand im Vakuumexsikkator vollständig getrocknet wurde.

Das aminomethylierte Zwischenprodukt, 2-Dimethylaminomethyl-4-ethyl-6-methoxyphenol, wurde als viskoses gelbliches Öl erhalten (Ausbeute: 19,43 g; 93,0 % d.Th.).

### Stufe 2:

### Variante 2.1:

2-Dimethylaminomethyl-4-ethyl-6-methoxyphenol (1,05 g, 5 mmol) wurde zusammen mit 1-Bromoctan (0,95 g, 4,9 mmol) und Tetrabutylammoniumbromid (TBAB) (0,16 g , 0,5 mmol) als Katalysator in 10 ml 2-Methyltetrahydrofuran (2-MeTHF) als Lösungsmittel bei Raumtemperatur heftig gerührt, bis eine homogene Lösung erhalten wurde, wonach festes gepulvertes KOH (0,56 g ,10 mmol) zugesetzt und 8 h lang bei Raumtemperatur gerührt wurde. Danach wurde der anorganische Feststoff abzentrifugiert und 3× mit 10 ml Diethylether gewaschen. Die vereinigten organischen Phasen wurden am Rotationsverdampfer eingeengt, der Rückstand erneut in 45 ml Petrolether gelöst und 4× mit je 5 ml Wasser gewaschen. Anschließend wurde die organische Phase am Rotationsverdampfer eingeengt und der Rückstand im Vakuumexsikkator vollständig getrocknet. Das veretherte Zwischenprodukt, N,N-Dimethyl-1-(5-ethyl-3-methoxy-2-octyloxyphenyl)methanamin, wurde als viskoses, gelbes Öl erhalten (Ausbeute: 1,45 g; 92,3 % d.Th.).

### Variante 2.2:

2-Dimethylaminomethyl-4-ethyl-6-methoxyphenol (1,05 g, 5 mmol) wurde zusammen mit 1-Bromoctan (1,06 g, 5,5 mmol) und Tetrabutylammoniumbromid (TBAB) (0,16 g , 0,5 mmol) in 10 ml 2-MeTHF bei Raumtemperatur heftig gerührt, bis eine homogene Lösung erhalten wurde, wonach festes gepulvertes KOH (0,56 g ,10 mmol) zugesetzt und 8 h lang bei Raumtemperatur gerührt wurde. Danach wurden 25 ml Et₂O und 5 ml H₂O zugesetzt, und die wässrige Phase wurde 3x mit 10 ml Et₂O extrahiert. Die vereinigten organischen Phasen wurden am Rotationsverdampfer eingeengt, und der Rückstand wurde über 5 g Kieselgel gelagert. Der Rückstand wurde mittels Flash-Chromatographie über eine Filtersäule gereinigt, wobei das veretherte Zwischenprodukt, N,N-Dimethyl-1-(5-ethyl-3-methoxy-2-octyloxyphenyl)methanamin, mit Et₂O eluierte und nach Abdampfen des Ethers als viskoses, gelbes Öl erhalten wurde (Ausbeute: 1,45 g; 92,9 % d.Th.).

### Stufe 3:

N,N-Dimethyl-1-(5-ethyl-3-methoxy-2-octyloxyphenyl)methanamin (0,96 g, 3 mmol) wurde vorgelegt, und 3 Äquivalente einer 30-gew.-%igen wässrigen Lösung von H₂O₂ (9 mmol) wurden auf einmal zugesetzt. Das trübe Reaktionsgemisch wurde bei Raumtemperatur über Nacht bzw. so lange gerührt, bis es klar und homogen erschien, was den vollständigen Verbrauch des Ausgangsmaterials anzeigte. Anschließend wurde eine katalytische Menge an Aktivkohle oder MnO₂ zugesetzt und das Gemisch 24 h lang gerührt, oder es wurde ofengetrocknetes Na₂CO₃ (1,06 g, 10 mmol) in 5 ml Ethanol zugesetzt und das Gemisch 30 min lang gerührt, um das überschüssige H₂O₂ zu zersetzen. Danach wurde der feste Niederschlag abzentrifugiert und 3x mit abs. EtOH gewaschen. Die vereinigten organischen Phasen wurden durch einen 0,2-µm-Spritzenfilter filtriert und anschließend am Rotationsverdampfer im Vakuum eingeengt, mit Hexan versetzt und erneut eingeengt, um das EtOH vollständig zu entfernen. Der Rückstand wurde danach im Vakuum vollständig getrocknet, wodurch die Titelverbindung (1) als klares, gelbes Öl erhalten wurde (Ausbeute: 0,98 g; 97,3 % d.Th.).

**¹H-NMR:** δ_{H} (600 MHz, CDCl₃) 6,90 (d, *J* = 2,0 Hz, 1H, C3), 6,81 (d, *J* = 2,0 Hz, 1H, C5), 4,54 (s, 2H, C12), 3,94 (t, *J* = 6,9, 6,9 Hz, 2H, C17), 3,86 (s, 3H, C11), 3,18 (s, 6H, C14, C15), 2,61 (q, *J* = 7,6, 7,6, 7,6 Hz, 2H, C7), 1,77 (p, *J* = 7,1, 7,1, 7,1, 7,1 Hz, 2H, C18), 1,41 (p, *J* = 7,1, 7,1, 6,8, 6,8 Hz, 2H, C19), 1,35-1,19 (m, 11H, C8, C20-23), 0,88 (t, *J* = 6,9, 6,9 Hz, 3H, C24). **¹³C-NMR**: δ_{C} (151 MHz, CDCl₃) δ 152,7 (C6), 146,0 (C1), 140,4 (C4), 124,6 (C3), 123,9 (C2), 114,0 (C5), 74,0 (C17), 70,5 (C12), 57,4 (C14, C15), 55,9 (C11), 32,0 (C18), 30,4 (C19), 29,5 (C20), 29,4 (C21), 28,7 (C7), 26,1 (C22), 22,8 (C23), 15,7 (C8), 14,2 (C24). **HRMS:** (ESI⁺, m/z) ber. für C₂₀H₃₆NO₃ [M+H]⁺: 338,26929; gef.: 338,26292.

### Beispiel 2

Herstellung von N,N-Dimethyl-1-(2-decyloxy-5-ethyl-3-methoxyphenyl)methanamin-N-oxid (2)

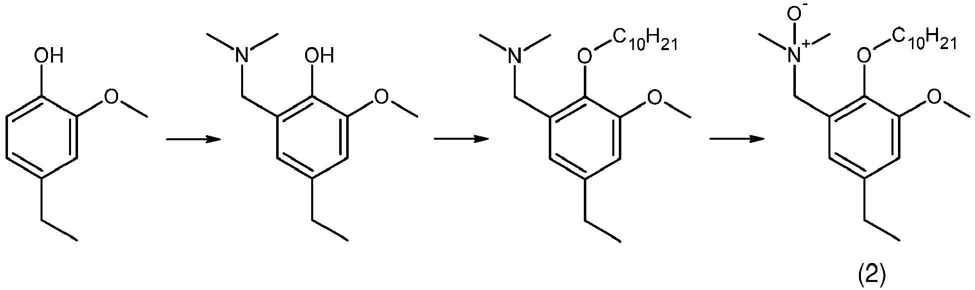

### Stufe 1:

Die Synthese und das Produkt waren identisch mit Beispiel 1.

### Stufe 2:

Die Reaktion erfolgte analog zu jener in Beispiel 1, außer dass 1-Bromdecan anstelle von 1-Bromoctan eingesetzt wurde, wobei N,N-Dimethyl-1-(2-decyloxy-5-ethyl-3-methoxyphenyl)methanamin als viskoses, gelbes Öl erhalten wurde.

### Variante 2.1:

Ausbeute: 1,50 g; 87,6 % d.Th.

### Variante 2.2:

Ausbeute: 1,63 g; 93,2 % d.Th.

### Stufe 3:

Die Reaktion erfolgte analog zu jener in Beispiel 1, außer dass nur 2 mmol N,N-Dimethyl-1-(2-decyloxy-5-ethyl-3-methoxyphenyl)methanamin eingesetzt wurden, wobei die Titelverbindung (2) als klares, gelbes Öl erhalten wurde (Ausbeute: 0,70 g; 96,1 % d.Th.).

**¹H-NMR:** δ_{H} (600 MHz, CDCl₃) 6,89 (d, *J* = 2,0 Hz, 1H, C3), 6,79 (d, *J* = 2,0 Hz, 1H, C5), 4,52 (s, 2H, C17), 3,93 (t, *J* = 6,9, 6,9 Hz, 2H, C18), 3,84 (s, 3H, C11), 3,16 (s, 6H, C14, C15), 2,60 (q, *J* = 7,6, 7,6, 7,6 Hz, 2H, C7), 1,76 (p, *J* = 7,1, 7,1, 7,1, 7,1 Hz, 2H, C18), 1,39 (p, *J* = 7,4, 7,4, 6,9, 6,9 Hz, 2H, C19), 1,34-1,17 (m, 16H, C8, C20-C25), 0,86 (t, *J* = 7,0, 7,0 Hz, 3H, C26). **¹³C-NMR**: δ_{C} (151 MHz, CDCl₃) 152,6 (C6), 145,9 (C1), 140,3 (C4), 124,5 (C3), 124,0 (C2), 113,9 (C5), 74,0 (C17), 70,5 (C12), 57,5 (C14, C15), 55,8 (C11), 32,0 (C18), 30,4 (C19), 29,7 (C20, C21), 29,5 (C22), 29,4 (C23), 28,7 (C7), 26,1 (C24), 22,8 (C25), 15,6 (C8), 14,2 (C26). **HRMS:** (ESI⁺, m/z) ber. für C₂₂H₄₀NO₃ [M+H]⁺: 366,30027; gef.: 366,30049.

### Beispiel 3

Herstellung von N,N-Dimethyl-1-(2-dodecyloxy-5-ethyl-3-methoxyphenyl)methan-amin-N-oxid (3)

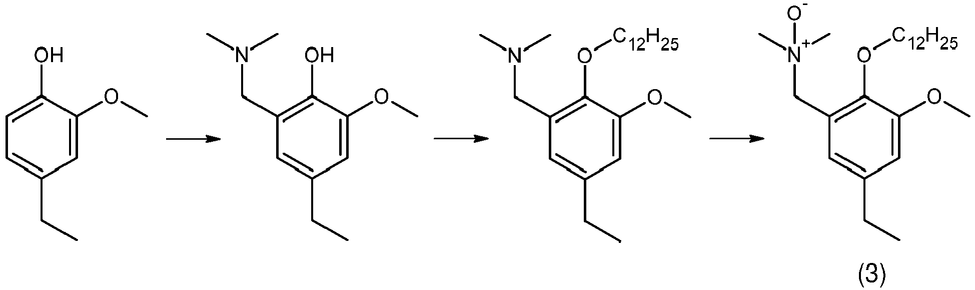

### Stufe 1:

Die Synthese und das Produkt waren identisch mit Beispiel 1.

### Stufe 2:

Die Reaktion erfolgte analog zu jener in Beispiel 1, außer dass 1-Bromdodecan anstelle von 1-Bromoctan eingesetzt wurde, wobei N,N-Dimethyl-1-(2-dodecyloxy-5-ethyl-3-methoxyphenyl)methanamin als viskoses, gelbes Öl erhalten wurde.

### Variante 2.1:

Ausbeute: 1,77 g; 96,0 % d.Th.

### Variante 2.2:

Ausbeute: 1,67 g; 88,4 % d.Th.

### Stufe 3:

Die Reaktion erfolgte analog zu jener in Beispiel 1, außer dass nur 2 mmol N,N-Dimethyl-1-(2-dodecyloxy-5-ethyl-3-methoxyphenyl)methanamin eingesetzt wurden, wobei die Titelverbindung (3) als klares, gelbes Öl erhalten wurde (Ausbeute: 0,75 g; 95,2 % d.Th.).

**¹H-NMR:** δ_{H} (600 MHz, CDCl₃) 6,80 (d, *J* = 2,0 Hz, 1H, C3), 6,64 (d, *J* = 2,0 Hz, 1H, C5), 3,88 (t, *J* = 6,7, 6,7 Hz, 2H, C17), 3,83 (s, 3H, C11), 3,45 (s, 2H, C12), 2,59 (q, *J* = 7,6, 7,6, 7,5 Hz, 2H, C7), 2,25 (s, 6H, C14, C15), 1,80-1,72 (m, 2H, C18), 1,47 (p, *J* = 7,3, 7,3, 7,0, 7,0 Hz, 2H, C19), 1,38-1,24 (m, 17H, C20-C27), 1,22 (t, *J* = 7,6, 7,6 Hz, 3H, C8), 0,88 (t, *J* = 7,0, 7,0 Hz, 3H, C28). **¹³C-NMR**: δ _{C} (151 MHz, CDCl₃) 152,6 (C6), 145,0 (C1), 139,7 (C4), 132,3 (C2), 121,6 (C3), 110,9 (C5), 73,5 (C17), 57,7 (C12), 55,9 (C11), 45,7 (C14, C15), 32,1 (C18), 30,5(C19), 29,8 (C20, C21), 29,8 (C22, C23), 29,7(C24), 29,5 (C25), 28,9 (C7), 26,3 (C26), 22,8 (C27), 15,8 (C8), 14,2 (C28). **Elementaranalyse:** Ber.: C, 76,34; H, 11,48; N, 3,71; gef.: C, 76,33; H, 11,64; N, 3,43; **HRMS:** (ESI⁺, m/z) ber. für C₂₄H₄₄NO₂ [M+H]⁺: 378,33666; gef.: 378,33690.

### Beispiel 4

Herstellung von N,N-Dimethyl-1-(5-ethyl-3-methoxy-2-tetradecyloxyphenyl)methan-amin-N-oxid (4)

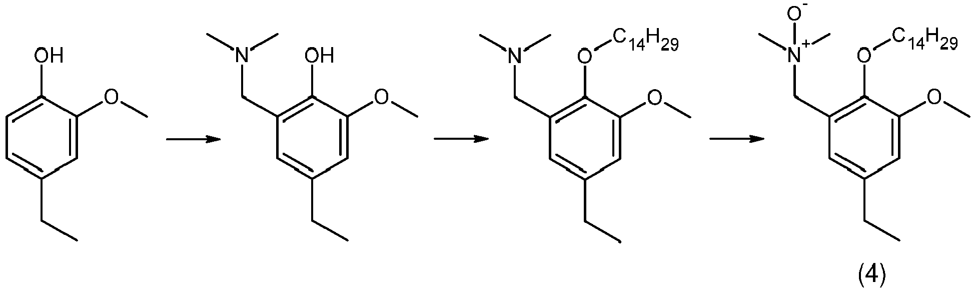

### Stufe 1:

Die Synthese und das Produkt waren identisch mit Beispiel 1.

### Stufe 2:

Die Reaktion erfolgte analog zu jener in Beispiel 1, außer dass 1-Bromtetradecan anstelle von 1-Bromoctan eingesetzt wurde, wobei N,N-Dimethyl-1-(5-ethyl-3-methoxy-2-tetradecyloxyphenyl)methanamin als viskoses, gelbes Öl erhalten wurde.

### Variante 2.1:

Ausbeute: 1,91 g; 96,1 % d.Th.

### Variante 2.2:

Ausbeute: 1,85 g; 91,1 % d.Th.

### Stufe 3:

Die Reaktion erfolgte analog zu jener in Beispiel 1, außer dass nur 2 mmol N,N-Dimethyl-1-(5-ethyl-3-methoxy-2-tetradecyloxyphenyl)methanamin eingesetzt wurden, wobei die Titelverbindung (4) als klares, gelbes Öl erhalten wurde (Ausbeute: 0,80 g; 94,3 % d.Th.).

**¹H-NMR:** δ_{H} (600 MHz, CDCl₃) 6,89 (d, *J* = 2,0 Hz, 1H, C3), 6,80 (d, *J* = 2,0 Hz, 1H, C5), 4,55 (s, 2H, C12), 3,93 (t, *J* = 6,9, 6,9 Hz, 2H, C17), 3,85 (s, 3H, C11), 3,19 (s, 6H, C14, C15), 2,60 (q, *J* = 7,6, 7,6, 7,6 Hz, 2H, C7), 1,76 (p, *J* = 7,1, 7,1, 7,1, 7,1 Hz, 2H, C18), 1,40 (p, *J* = 7,3, 7,3, 6,9, 6,9 Hz, 2H, C19), 1,34-1,19 (m, 23H, C18, C20-C29), 0,86 (t, *J* = 7,0, 7,0 Hz, 3H, C30). **¹³C-NMR**: δ_{C} (151 MHz, CDCl₃) 152,6 (C6), 145,9 (C1), 140,4 (C4), 124,6 (C3), 123,7 (C2), 114,0 (C5), 74,0 (C17), 70,2 (C12), 57,2 (C14, C15), 55,9 (C11), 32,0 (C18), 30,4 (C19), 29,8 (C20, C21), 29,8 (C22, C23), 29,8 (C24), 29,7 (C25), 29,5 (C26), 29,5 (C27), 28,7 (C7), 26,1 (C28), 22,8 (C29), 15,7 (C8), 14,2 (C30). **HRMS:** (ESI⁺, m/z) ber. für C₂₆H₄₈NO₃ [M+H]⁺: 422,36287; gef.: 422,36323.

### Beispiel 5

Herstellung von N,N-Dimethyl-1-(5-ethyl-2-hexadecyloxy-3-methoxyphenyl)methan-amin-N-oxid (5)

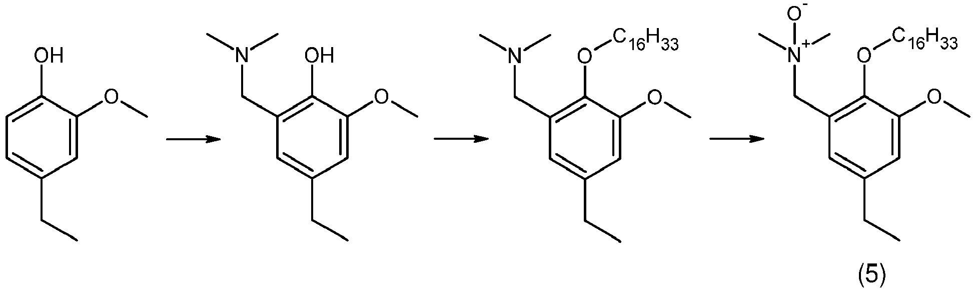

### Stufe 1:

Die Synthese und das Produkt waren identisch mit Beispiel 1.

### Stufe 2:

Die Reaktion erfolgte analog zu jener in Beispiel 1, außer dass 1-Bromhexadecan anstelle von 1-Bromoctan eingesetzt wurde, wobei N,N-Dimethyl-1-(5-ethyl-2-hexadecyl-oxy-3-methoxyphenyl)methanamin als viskoses, gelbes Öl erhalten wurde.

### Variante 2.1:

Ausbeute: 2,00 g; 94,0 % d.Th.

### Variante 2.2:

Ausbeute: 2,11 g; 97,4 % d.Th.

### Stufe 3:

Die Reaktion erfolgte analog zu jener in Beispiel 1, außer dass nur 2 mmol N,N-Dimethyl-1-(5-ethyl-2-hexadecyloxy-3-methoxyphenyl)methanamin eingesetzt wurden, wobei die Titelverbindung (5) als klares, gelbes Öl erhalten wurde (Ausbeute: 0,87 g; 97,0 % d.Th.).

**¹H-NMR:** δ_{H} (600 MHz, CDCl₃) 6,90 (d, *J* = 1,9 Hz, 1H, C3), 6,81 (d, *J* = 1,9 Hz, 1H, C5), 4,53 (s, 2H, C11), 3,94 (t, *J* = 6,9, 6,9 Hz, 2H, C17), 3,86 (s, 3H, C11), 3,18 (s, 6H, C14, C15), 2,62 (q, *J* = 7,6, 7,6, 7,6 Hz, 2H, C7), 1,77 (p, *J* = 7,1, 7,1, 7,1, 7,1 Hz, 2H, C18), 1,41 (p, *J* = 7,3, 7,3, 6,9, 6,9 Hz, 2H, , C19), 1,36-1,20 (m, 29H, C8, C20-C31), 0,87 (t, *J* = 6,9, 6,9 Hz, 3H, C32). **¹³C-NMR**: δ_{C} (151 MHz, CDCl₃) 152,7 (C6), 146,0 (C1), 140,4 (C4), 124,5 (C3), 123,9 (C2), 114,0 (C5), 74,0 (C17), 70,5 (C12), 57,5 (C14, C15), 55,9 (C11), 32,1 (C18), 30,4 (C19), 29,8 (C20), 29,8 (C21, C22), 29,8 (C23, C24), 29,8 (C25), 29,7 (C26), 29,6 (C27), 29,5 (C28), 28,7 (C29) (C7), 26,1 (C30), 22,8 (C31), 15,7 (C8), 14,3 (C32). **HRMS:** (ESI⁺, m/z) ber. für C₂₈H₅₂NO₃ [M+H]⁺: 450,39417; gef.: 450,39428.

### Beispiel 6

Herstellung von N,N-Dimethyl-1-(5-ethyl-3-methoxy-2-octadecyloxyphenyl)methan-amin-N-oxid (6)

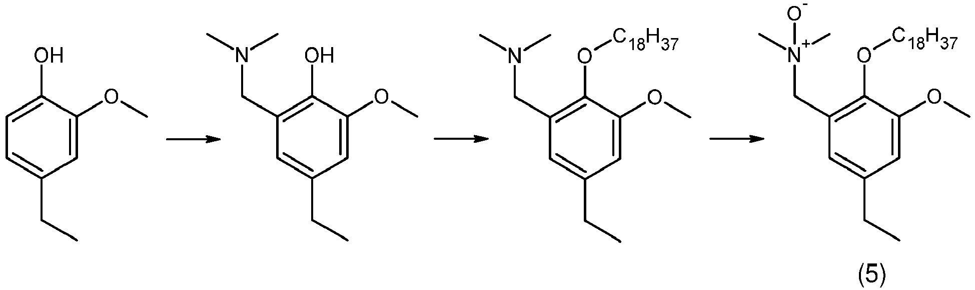

### Stufe 1:

Die Synthese und das Produkt waren identisch mit Beispiel 1.

### Stufe 2:

Die Reaktion erfolgte analog zu jener in Beispiel 1, außer dass 1-Bromoctadecan anstelle von 1-Bromoctan eingesetzt wurde, wobei N,N-Dimethyl-1-(5-ethyl-3-methoxy-2-octadecyloxyphenyl)methanamin als viskoses, gelbes Öl (Variante 2.1; Ausbeute: 2,06 g; 91,0 % d.Th.) bzw. als weißer, wachsartiger Feststoff (Variante 2.2; Ausbeute: 2,00 g; 86,3 % d.Th.) erhalten wurde.

### Stufe 3:

Die Reaktion erfolgte analog zu jener in Beispiel 1, außer dass nur 2 mmol N,N-Dimethyl-1-(5-ethyl-3-methoxy-2-octadecyloxyphenyl)methanamin eingesetzt und 0,5 ml MeOH zur Erhöhung der Löslichkeit zugesetzt wurden, wobei die Titelverbindung (6) als weißer, wachsartiger Feststoff erhalten wurde (Ausbeute: 0,916 g; 95,9 % d.Th.).

**¹H-NMR:** δ_{H} (600 MHz, CDCl₃) 6,91 (d, *J* = 2,0 Hz, 1H, C3), 6,82 (d, *J* = 2,0 Hz, 1H, C5), 4,56 (s, 2H, C12), 3,95 (t, *J* = 6,9, 6,9 Hz, 2H, C17), 3,86 (s, 3H, C11), 3,19 (s, 6H, C14, C15), 2,62 (q, *J* = 7,6, 7,6, 7,6 Hz, 2H, C7), 1,77 (p, *J* = 7,1, 7,1, 7,1, 7,1 Hz, 2H, C18), 1,41 (p, *J* = 7,2, 7,2, 6,9, 6,9 Hz, 2H, C19), 1,36-1,19 (m, 30H, C8, C20-C33), 0,87 (t, *J* = 7,0, 7,0 Hz, 3H, C34). **¹³C-NMR**: δ_{C} (151 MHz, CDCl₃) 152,7 (C6), 146,0 (C1), 140,4 (C4), 124,5 (C3), 123,7 (C2), 114,0 (C5), 74,0 (C17), 70,3 (C12), 57,3 (C14, C15), 55,9 (C11), 32,1 (C18), 30,4 (C19), 29,8 (C20, C21, C22, C23), 29,8 (C24, C25, C26, C27), 29,8 (C28), 29,7 (C29), 29,6 (C30), 29,5 (C31), 28,7 (C7), 26,1 (C32), 22,8 (C33), 15,7 (C8), 14,3 (C34). **HRMS:** (ESI⁺, m/z) ber. für C₃₀H₅₆NO₃ [M+H]⁺: 478,42547; gef.: 478,42540.

### Beispiel 7

Herstellung von 1-(2-Dodecyloxy-5-ethyl-3-methoxybenzyl)pyrrolidin-1-oxid (7)

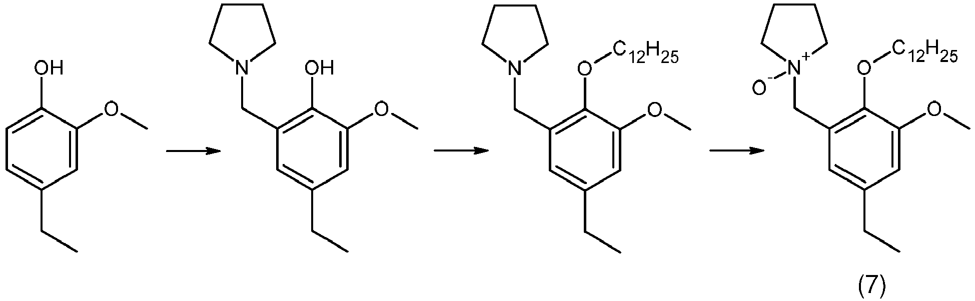

### Stufe 1:

Eine wässrige Lösung von 4-Ethylguajacol (4,56 g, 30 mmol) wurde binnen 15 min unter konstantem Rühren zu einer wässrigen Lösung (10 ml) von Pyrrolidin (2,21 g, 31 mmol) in einem Eiswasserbad zugetropft. Dazu wurde Paraformaldehyd (1,35 g, 45 mmol) in Aliquoten von jeweils 0,5 g alle 10 min zugesetzt und 3 h lang im Eiswasserbad und danach 72 h lang bei Raumtemperatur gerührt. Anschließend wurde das Reaktionsgemisch 5x mit 25 ml Petrolether extrahiert, die vereinigten organischen Phasen 5x mit 4 ml Wasser gewaschen und am Rotationsverdampfer im Vakuum eingeengt, wonach der Rückstand im Vakuumexsikkator vollständig getrocknet wurde. Das aminomethylierte Zwischenprodukt, 4-Ethyl-6-methoxy-2-(pyrrolidinomethyl)-phenol, wurde als viskoses, hellgelbes Öl erhalten (Ausbeute: 6,50 g; 92,1 % d.Th.).

### Stufe 2:

Die Reaktion erfolgte analog zu jener in Beispiel 1, Variante 2.1, außer dass 1-Bromdodecan anstelle von 1-Bromoctan eingesetzt wurde, wobei 1-(2-Dodecyloxy-5-ethyl-3-methoxybenzyl)pyrrolidin als leicht gelbliches Öl erhalten wurde (Ausbeute: 1,93 g; 97,6 % d.Th.).

### Stufe 3:

Die Reaktion erfolgte analog zu jener in Beispiel 1, außer dass nur 2 mmol 1-(2-Dodecyloxy-5-ethyl-3-methoxybenzyl)pyrrolidin eingesetzt wurden, wobei die Titelverbindung (7) als klares, gelbes Öl erhalten wurde (Ausbeute: 0,80 g; 95,5 % d.Th.).

**¹H-NMR:** δ_{H} (300 MHz, CDCl₃) 6,94 (d, *J* = 2,0 Hz, 1H, 2), 6,76 (d, *J* = 2,0 Hz, 1H, 4), 4,64 (s, 2H, 12), 3,91 (t, *J* = 6,9 Hz, 2H, 19), 3,83 (s, 3H, 9), 3,50-3,21 (m, 4H, 14', 17'), 2,58 (q, *J* = 7,6 Hz, 2H, 10), 2,49-2,34 (m, 2H, 20), 1,87-1,69 (m, 4H, 15", 16"), 1,37-1,15 (m, 21H, 11, 21, 22, 23, 24, 25, 26, 27, 28, 29), 0,85 (t, *J* = 6,9 Hz, 3H, 30). **¹³C-NMR:** δ_{C} (75 MHz, CDCl₃) 152,5 (5), 145,7 (6), 140,2 (3), 125,1 (2), 124,3 (1), 113,4 (4), 73,9 (19), 65,5 (14, 17), 65,5, (12), 55,8 (9), 32,0 (20), 30,3 (21), 29,7 (22, 23), 29,7 (24, 25), 29,7 (26), 29,5 (27), 29,4 (28), 28,6 (10), 26,1, 22,8 , (15, 16), 21,3 (29), 15,6 (11), 14,2 (30). **HRMS:** (ESI⁺, m/z) ber. für C₂₆H₄₆NO₃ [M+H]⁺: 420,34777; gef.: 420,347257.

### Beispiel 8

Herstellung von 1-(2-Dodecyloxy-5-ethyl-3-methoxybenzyl)piperidin-1-oxid (8)

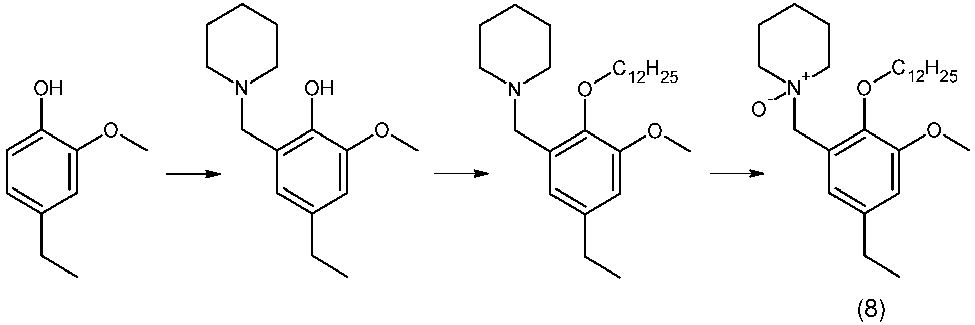

### Stufe 1:

Die Reaktion erfolgte analog zu jener in Beispiel 7, außer dass Piperidin (2,64 g, 31 mmol) anstelle von Pyrrolidin eingesetzt wurde, wobei 4-Ethyl-6-methoxy-2-(piperidinomethyl)phenol als leicht gelbliches Öl erhalten wurde (Ausbeute: 7,06 g; 94,4 % d.Th.).

### Stufe 2:

Die Reaktion erfolgte analog zu jener in Beispiel 1, Variante 2.1, außer dass 10 mmol 4-Ethyl-6-methoxy-2-(piperidinomethyl)phenol und 1-Bromdodecan statt 1-Bromoctan eingesetzt wurden, wobei 1-(2-Dodecyloxy-5-ethyl-3-methoxybenzyl)piperidin als leicht gelbliches Öl erhalten wurde (Ausbeute: 3,93 g; 94,1 % d.Th.).

### Stufe 3:

Die Reaktion erfolgte analog zu jener in Beispiel 1, außer dass 5 mmol 1-(2-Dodecyloxy-5-ethyl-3-methoxybenzyl)piperidin eingesetzt wurden, wobei die Titelverbindung (8) als klares, gelbes Öl erhalten wurde (Ausbeute: 2,10 g; 96,8 % d.Th.).

**¹H-NMR:** δ_{H} (300 MHz, CDCl₃) 6,99 (d, *J* = 2,0 Hz, 1H), 6,79 (d, *J* = 2,0 Hz, 1H), 4,50 (s, 2H), 3,92 (t, *J* = 6,9 Hz, 2H), 3,85 (s, 3H), 3,29 (d, *J* = 11,8 Hz, 2H), 3,04 (td, *J* = 12,2, 3,1 Hz, 2H), 2,61 (q, *J* = 7,6 Hz, 2H), 2,47-2,26 (m, 2H), 1,85-1,02 (m, 28H), 0,87 (t, *J* = 7,0, 6,2 Hz, 3H). **¹³C-NMR**: δ _{C} (75 MHz, CDCl₃) 152,4, 146,0, 140,1, 125,3, 123,5, 113,5, 74,0, 70,6, 63,7, 55,8, 32,0, 30,4, 29,8, 29,8, 29,8, 29,6, 29,5, 28,7, 26,2, 22,8, 22,0, 20,6, 15,6, 14,2. **HRMS:** (ESI⁺, m/z) ber. für C₂₇H₄₈NO₃ [M+H]⁺: 434,36342; gef.: 434,362833.

### Beispiel 9

Herstellung von 1-(2-Dodecyloxy-5-ethyl-3-methoxybenzyl)-4-methylpiperazin-1,4-dioxid (9)

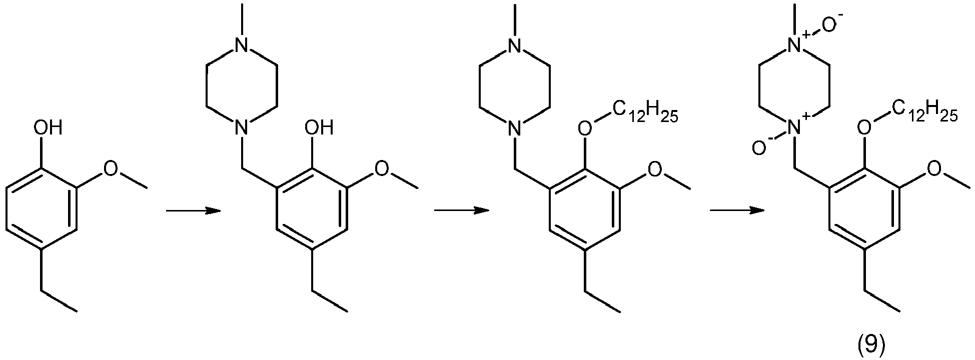

### Stufe 1:

Eine wässrige Lösung von 4-Ethylguajacol (3,04 g, 20 mmol) wurde binnen 15 min unter konstantem Rühren zu einer wässrigen Lösung (5 ml) von 1-Methylpiperazin (2,64 g, 30 mmol) in einem Eiswasserbad zugetropft. Dazu wurde eine 37-gew.-%ige wässrige Formaldehyd-Lösung (0,90 g, 30 mmol) in Aliquoten von jeweils 0,1 g alle 10 min zugesetzt und 3 h lang im Eiswasserbad und danach 9 h lang bei Raumtemperatur gerührt. Anschließend wurde der ausgefallene Feststoff abzentrifugiert und erneut in 45 ml Et₂O gelöst. Die Lösung wurde 5x mit 5 ml Wasser gewaschen und danach am Rotationsverdampfer im Vakuum eingeengt, wobei das aminomethylierte Zwischenprodukt, 4-Ethyl-6-methoxy-2-(4-methylpiperazinomethyl)phenol, als weißes Pulver erhalten wurde (Ausbeute: 1,65 g; 31,1 % d.Th.).

### Stufe 2:

Die Reaktion erfolgte analog zu jener in Beispiel 1, Variante 2.2, außer dass 1-Bromdodecan statt 1-Bromoctan eingesetzt wurde, wobei 1-(2-Dodecyloxy-5-ethyl-3-methoxybenzyl)-4-methylpiperazin als cremefarbenes Pulver erhalten wurde (Ausbeute: 0,28 g; 12,9 % d.Th.).

### Stufe 3:

Die Reaktion erfolgte analog zu jener in Beispiel 1, außer dass 0,125 mmol 1-(2-Dodecyloxy-5-ethyl-3-methoxybenzyl)-4-methylpiperazin eingesetzt wurden, wobei die Titelverbindung (9) als cremefarbenes Pulver erhalten wurde (Ausbeute: 0,058 g; 99,9 % d.Th.).

**¹H-NMR:** δ_{H} (300 MHz, CDCl₃) 6,97 (d, *J* = 2,0 Hz, 1H), 6,79 (d, *J* = 2,0 Hz, 1H), 4,46 (s, 2H), 4,39-4,18 (m, 4H), 3,95 (t, *J* = 7,0, 7,0 Hz, 2H), 3,83 (s, 3H), 3,25 (s, 3H), 3,01 (dd, *J* = 15,4, 9,6 Hz, 4H), 2,60 (q, *J* = 7,6, 7,6, 7,6 Hz, 2H), 1,81-1,68 (m, 2H), 1,45-1,13 (m, 22H), 0,85 (d, *J* = 6,8 Hz, 3H). **¹³C-NMR**: δ_{C} (75 MHz, CDCl₃) 152,2, 145,8, 140,3, 125,4, 121,6, 114,4, 74,0, 69,3, 60,0, 59,1, 57,8, 55,8, 32,0, 29,9, 29,8, 29,8, 29,7, 29,5, 29,4, 28,6, 25,8, 22,8, 15,5, 14,2. **HRMS:** (ESI⁺, m/z) ber. für C₂₇H₄₈N₂O₄ [M+H]⁺: 465,368684; gef.: 465,367767.

### Beispiel 10

Herstellung von N,N-Dimethyl-N'-(2-dodecyloxy-5-ethyl-3-methoxybenzyl)-N'-methyl-ethan-1,2-diamin-di-N-oxid (10)

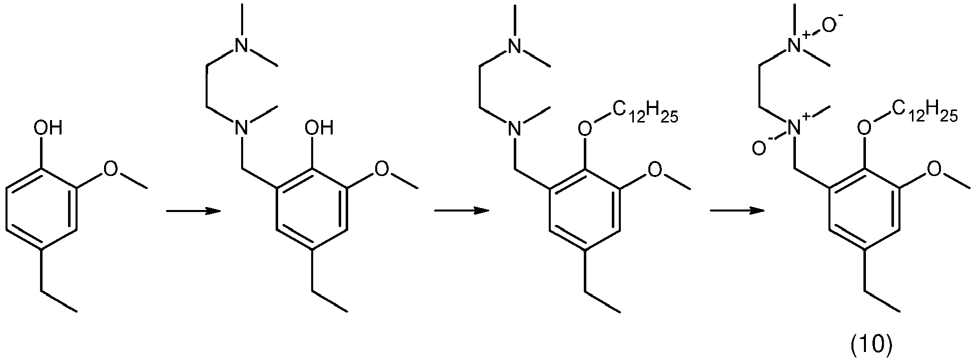

### Stufe 1:

Die Reaktion erfolgte analog zu jener in Beispiel 7, außer dass N,N,N'-Trimethylethan-1,2-diamin (3,07 g, 30 mmol) anstelle von Pyrrolidin eingesetzt wurde, wobei 2-(2-Dimethylaminoethyl)aminomethyl-4-ethyl-6-methoxyphenol als cremefarbenes Pulver erhalten wurde (Ausbeute: 4,59 g; 86,3 % d.Th.).

### Stufe 2:

Die Reaktion erfolgte analog zu jener in Beispiel 1, Variante 2.2, außer dass 1-Bromdodecan statt 1-Bromoctan eingesetzt wurde, wobei N,N-Dimethyl-N'-(2-Dodecyloxy-5-ethyl-3-methoxybenzyl)-N'-methylethan-1,2-diamin als cremefarbenes Pulver erhalten wurde (Ausbeute: 1,17 g; 53,8 % d.Th.).

### Stufe 3:

Die Reaktion erfolgte analog zu jener in Beispiel 1, außer dass 1 mmol N,N-Dimethyl-N'-(2-Dodecyloxy-5-ethyl-3-methoxybenzyl)-N'-methylethan-1,2-diamin eingesetzt wurde, wobei die Titelverbindung (10) als cremefarbenes Pulver erhalten wurde (Ausbeute: 0,97 g; 69,0 % d.Th.).

¹H-NMR δ_{H} (300 MHz, CDCl₃) 6,95 (d, *J* = 2,0 Hz, 1H), 6,80 (d, *J* = 2,0 Hz, 1H), 4,49 (d, *J* = 12,4 Hz, 1H), 4,36 (d, *J* = 12,4 Hz, 1H), 4,07-3,92 (m, 2H), 3,91-3,79 (m, 3+3H), 3,67 (q, *J* = 7,0, 7,0, 7,0 Hz, 1H), 3,33-3,17 (m, 6H), 3,04 (s, 3H), 2,70-2,56 (m, 2H), 1,75 (dd, *J* = 10,8, 4,7 Hz, 2H), 1,45-1,35 (m, 2H), 1,35-1,17 (m, 28H), 0,85 (t, *J* = 6,9 Hz, 3H). ¹³C-NMR δ_{C} (75 MHz, CDCl₃) 152,6, 146,0, 140,4, 124,2, 114,1, 74,0, 64,6, 55,9, 32,0, 30,4, 29,8, 29,7, 29,6, 29,5, 28,7, 26,1, 22,8, 15,7, 14,2.

### Beispiel 11

Herstellung von 1,1'-(2,3-Dioctyloxy-5-ethyl-1,4-phenylen)-bis(N,N-dimethylmethan-amin-N-oxid) und 1,1'-(2,3-Dioctyloxy-5-ethyl-1,6-phenylen)-bis(N,N-dimethylmethan-amin-N-oxid) (11)

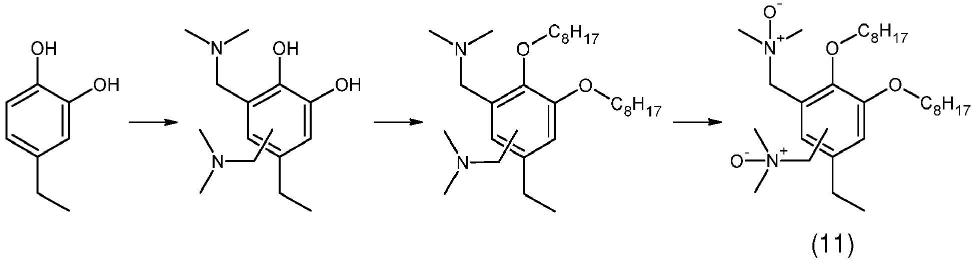

### Stufe 1:

Eine wässrige Lösung von 4-Ethylbrenzcatechin (1,0 g, 7,24 mmol) wurde binnen 15 min unter konstantem Rühren und unter Argon-Atmosphäre zu einer 40-gew.-%-igen wässrigen Lösung von Dimethylamin (0,98 g, 21,7 mmol) in einem Eiswasserbad zugetropft. Dazu wurden 10 ml einer 37-gew.-%igen wässrigen Formaldehyd-Lösung (0,65 g, 21,7 mmol) in 5 Aliquoten alle 10 min zugesetzt und 2 h lang im Eiswasserbad und danach 58 h lang bei Raumtemperatur gerührt. Anschließend wurde das Reaktionsgemisch 5x mit 10 ml Et₂O extrahiert, und die vereinigten organischen Phasen wurden 5x mit 5 ml Wasser gewaschen und danach am Rotationsverdampfer im Vakuum eingeengt, wonach der Rückstand im Vakuumexsikkator vollständig getrocknet wurde, wobei ein Gemisch der doppelt aminomethylierten Zwischenprodukte, 3,4- und 3,6-Bis(dimethylaminomethyl)-5-ethylbrenzcatechin, als cremefarbenes Pulver erhalten wurde (Ausbeute: 1,20 g; 65,5 % d.Th.).

### Stufe 2:

Die Reaktion erfolgte analog zu jener in Beispiel 1, Variante 2.2, außer dass sie unter Argon-Atmosphäre 12 h lang durchgeführt wurde und bei der Flash-Chromatographie die Elution mit einem Petrolether/Ethylacetat-Gradienten erfolgte, wobei ein Gemisch aus 1,1'-(2,3-Dioctyloxy-5-ethyl-1,4-phenylen)-bis(N,N-dimethylmethanamin) und 1,1'-(2,3-Dioctyloxy-5-ethyl-1,6-phenylen)-bis(N,N-dimethylmethanamin) als gelbes Öl erhalten wurde (Ausbeute: 0,15 g; 12,3 % d.Th.).

**¹H-NMR:** δ_{H} (300 MHz, CDCl₃) δ 6,91 (s, 1H), 3,92 (dt, *J* = 8,2, 6,7 Hz, 4H), 3,40 (d, *J* = 7,1 Hz, 4H), 2,71 (q, *J* = 7,5 Hz, 2H), 2,24 (d, *J* = 6,6 Hz, 12H), 1,75 (dt, *J* = 8,3, 6,3 Hz, 4H), 1,53-1,39 (m, 4H), 1,36-1,25 (m, 18H), 1,19 (t, *J* = 7,5 Hz, 3H), 0,94-0,83 (m, 6H). **¹³C-NMR**: δ_{C} (75 MHz, CDCl₃) 151,5, 148,9, 139,9, 131,4, 129,7, 125,3, 73,4 (d, *J* = 2,2 Hz), 58,2, 54,2, 45,7, 32,0, 30,7 (d, *J* = 2,0 Hz), 29,7, 29,5, 26,4 (d, *J* = 1,5 Hz), 25,3, 22,8, 15,7, 14,3. **Elementaranalyse:** Ber.: C, 75,57; H, 11,84; N, 5,88; gef.: C, 75,34; H, 11,85; N, 5,56; **HRMS:** (ESI⁺, m/z) ber. für C₃₀H₅₇N₂O₂ [M+H]⁺: 477,4420; gef.: 477,441284.

### Stufe 3:

Die Reaktion erfolgte analog zu jener in Beispiel 1, außer dass 0,25 mmol des Amin-Gemischs eingesetzt wurden, wobei jedoch die Titelverbindungen (11) aufgrund von Nebenreaktionen im Gemisch mit mehreren Nebenprodukten anfielen. Die Optimierung dieser Oxidationsreaktion ist derzeit Gegenstand der Forschung der Erfinder.

### Beispiel 12

Herstellung von 1,1'-(5-Ethyl-2-octyloxy-1,3-phenylen)-bis(N,N-dimethylmethanamin-N-oxid) (12)

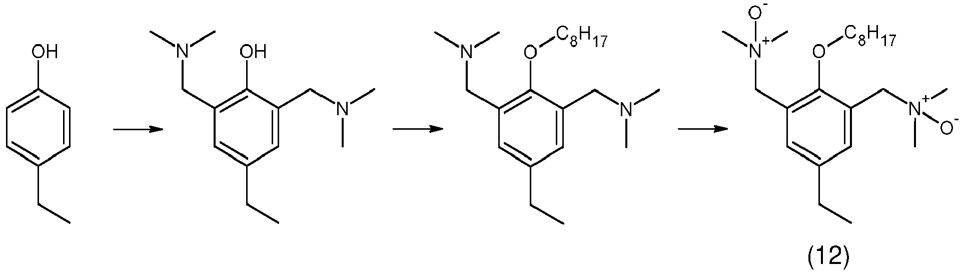

### Stufe 1:

Eine wässrige Lösung von 4-Ethylphenol (6,11 g, 50 mmol) wurde binnen 15 min unter konstantem Rühren und zu einer 40-gew.-%igen wässrigen Lösung von Dimethylamin (6,76 g, 150 mmol) in einem Eiswasserbad zugetropft. Dazu wurde Paraformaldehyd (4,50 g, 150 mmol) in Aliquoten von jeweils 1,5 g alle 10 min zugesetzt und 2 h lang im Eiswasserbad und danach 58 h lang bei Raumtemperatur gerührt. Anschließend wurde das Reaktionsgemisch 5x mit 25 ml Petrolether extrahiert, und die vereinigten organischen Phasen wurden 5x mit 4 ml Wasser gewaschen und am Rotationsverdampfer im Vakuum eingeengt, wonach der Rückstand im Vakuumexsikkator vollständig getrocknet wurde. Das doppelt aminomethylierte Zwischenprodukt, 2,6-Bis-(dimethylaminomethyl)-4-ethylphenol, wurde als viskoses klares Öl erhalten (Ausbeute: 11,02 g; 93,3 %d.Th.).

### Stufe 2:

Die Reaktion erfolgte analog zu jener in Beispiel 1, Variante 2.2, außer dass das KOH in zwei Portionen (je 0,28 g zu Beginn und nach 2 h) zugesetzt wurde und bei der Flash-Chromatographie die Elution mit einem Petrolether/Ethylacetat-Gradienten erfolgte, wobei 1,1'-(5-Ethyl-2-octyloxy-1,3-phenylen)-bis(N,N-dimethylmethanamin) als gelbes Öl erhalten wurde (Ausbeute: 1,54 g; 8,6 % d.Th.).

### Stufe 3:

Die Reaktion erfolgte analog zu jener in Beispiel 1, wobei die Titelverbindung (12) als viskoses, klares, gelbes Öl erhalten wurde (Ausbeute: 1,12 g; 97,9 % d.Th.).

**¹H-NMR:** δ_{H} (300 MHz, CDCl₃) 7,51 (s, 2H, C2, C4), 4,48 (s, 4H, C8, C11), 3,71 (t, *J* = 6,8, 6,8 Hz, 2H, C19), 3,16 (s, 12H, C13, C14, C16, C17), 2,67-2,54 (m, 2H, C9), 1,89-1,76 (m, 2H, C20), 1,45-1,34 (m, 2H, C21), 1,33-1,04 (m, 14H, C10, C22-C25), 0,85 (t, *J* = 6,7 Hz, 3H, C26). **¹³C-NMR**: δ_{C} (75 MHz, CDCl₃) 156,4 (C6), 141,1 (C3), 136,7 (C1, C5), 124,0 (C2, C4), 69,4 (C8, C11), 57,6 (C13, C14, C16, C17), 31,9 (C20), 30,4 (C21), 29,5 (C22), 29,3 (C23), 28,0 (C9), 26,2 (C24), 22,7 (C25), 15,3 (C10), 14,2 (C26). **HRMS:** (ESI⁺, m/z) ber. für C₂₂H₄₁NO₃ [M+H]⁺: 381,31172; gef.: 381,310922.

### Beispiel 13

Herstellung von 1,1'-(2-Dodecyloxy-5-ethyl-1,3-phenylen)-bis(N,N-dimethylmethan-amin-N-oxid) (13)

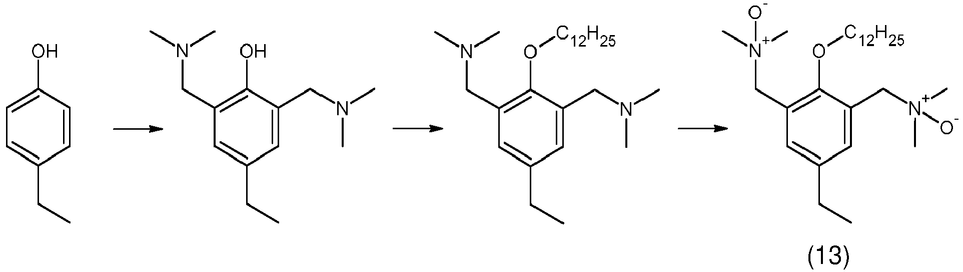

### Stufe 1:

Die Synthese und das Produkt waren identisch mit Beispiel 12.

### Stufe 2:

Die Reaktion erfolgte analog zu jener in Beispiel 1, Variante 2.2, außer dass 1-Bromdodecan anstelle von 1-Bromoctan eingesetzt wurde, das KOH in zwei Portionen (je 0,28 g zu Beginn und nach 2 h) zugesetzt wurde und bei der Flash-Chromatographie die Elution mit einem Petrolether/Ethylacetat-Gradienten erfolgte, wobei 1,1'-(2-Dodecyloxy-5-ethyl-1,3-phenylen)-bis(N,N-dimethylmethanamin) als gelbes Öl erhalten wurde (Ausbeute: 1,53 g; 75,7 % d.Th.).

### Stufe 3:

Die Reaktion erfolgte analog zu jener in Beispiel 1, außer dass nur 2,5 mmol 1,1'-(2-Dodecyloxy-5-ethyl-1,3-phenylen)-bis(N,N-dimethylmethanamin) eingesetzt wurden, wobei die Titelverbindung (13) als cremefarbener, wachsartiger Feststoff erhalten wurde (Ausbeute: 0,70 g; 96,1 % d.Th.).

**¹H-NMR:** δ_{H} (300 MHz, CDCl₃) 7,53 (s, 2H, C2, C4), 4,49 (s, 4H, C8, C11), 3,74 (t, J = 6,8 Hz, 2H, C19), 3,19 (s, 12H, C13, C14, C16, C17), 2,64 (q, J = 7,6 Hz, 2H, C9), 1,84 (t, J = 7,4 Hz, 2H, C20), 1,46-1,37 (m, 2H, C21), 1,32-1,17 (m, 22H, C10, C22-C29), 0,87 (t, J = 6,9 Hz, 3H, C30). **¹³C-NMR**: δ_{C} (75 MHz, CDCl₃) 156,4 (C6), 141,2 (C3), 136,8 (C1, C5), 123,9 (C2, C4), 69,4 (C8, C11), 57,6 (C13, C14, C16, C17), 32,0 (C20), 30,5 (C21), 29,8 (C22, C24, C25), 29,6 (C26), 29,5 (C27), 28,1 (C9), 26,3 (C28), 22,8 (C29), 15,4 (C10), 14,3 (C30). **HRMS:** (ESI+, m/z) ber. für C₂₆H₄₉NO₃ [M+H]⁺: 437,37432; gef.: 437,373256.

### Beispiel 14

Herstellung von 1,1'-(5-Ethyl-2-hexadecyloxy-1,3-phenylen)-bis(N,N-dimethylmethan-amin-N-oxid) (14)

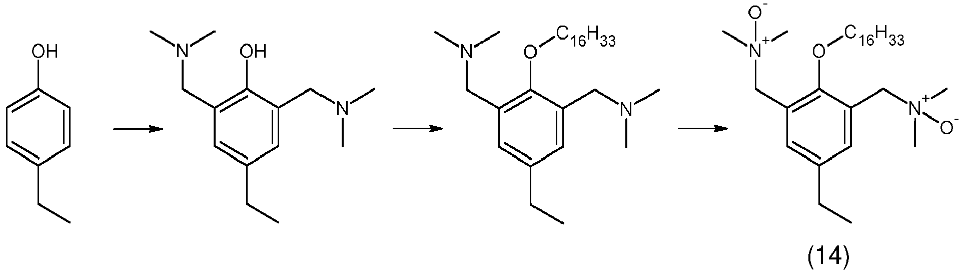

### Stufe 1:

Die Synthese und das Produkt waren identisch mit Beispiel 12.

### Stufe 2:

Die Reaktion erfolgte analog zu jener in Beispiel 13, außer dass 1-Bromhexadecan anstelle von 1-Bromdodecan eingesetzt wurde, wobei 1,1'-(5-Ethyl-2-hexadecyloxy-1,3-phenylen)-bis(N,N-dimethylmethanamin) als gelbes Öl erhalten wurde (Ausbeute: 1,57 g; 68,1 % d.Th.).

### Stufe 3:

Die Reaktion erfolgte analog zu jener in Beispiel 13, wobei die Titelverbindung (14) als cremefarbener, wachsartiger Feststoff erhalten wurde (Ausbeute: 1,20 g; 97,2 % d.Th.).

**¹H-NMR:** δ_{H} (300 MHz, CDCl₃) 7,54 (s, 2H, C2, C4), 4,50 (s, 4H, C8, C11), 3,74 (t, *J* = 6,8 Hz, 2H, C19), 3,17 (s, 12H, C13, C14, C16, C17), 2,65 (q, *J* = 7,5 Hz, 2H, C9), 1,88-1,82 (m, 2H, C20), 1,48-1,39 (m, 2H, C21), 1,30-1,21 (m, 29H, C10, C22-C33), 0,86 (t, *J* = 7,0 Hz, 3H, C34). **¹³C-NMR**: δ_{C} (75 MHz, CDCl₃) 156,4 (C6), 141,2 (C3), 136,7 (C1, C5), 124,2 (C2, C4), 69,6 (C8, C11), 57,8 (C13, C14, C16, C17), 32,0 (C20), 30,5 (C21), 30,0-29,3 (m) (C22-C31), 28,1 (C9), 26,3 (C32), 22,8 (C33), 15,4 (C10), 14,3 (C34). **HRMS:** (ESI⁺, m/z) ber. für C₃₀H₅₇N₂O₃ [M+H]⁺: 493,43692; gef.: 493,436311.

### Beispiel 15

Herstellung von 1,1'-(5-Ethyl-2-octadecyloxy-1,3-phenylen)-bis(N,N-dimethylmethan-amin-N-oxid) (15)

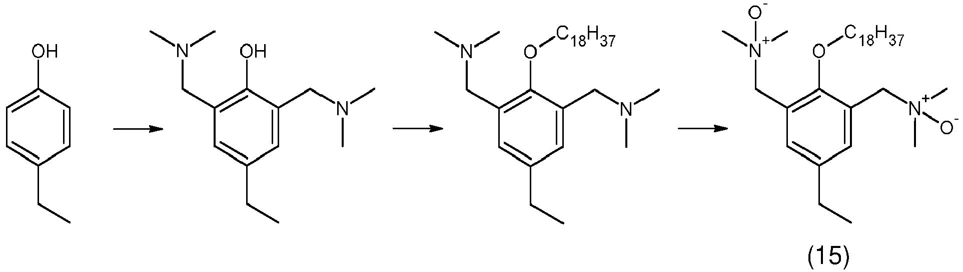

### Stufe 1:

Die Synthese und das Produkt waren identisch mit Beispiel 12.

### Stufe 2:

Die Reaktion erfolgte analog zu jener in Beispiel 13, außer dass 1-Bromoctadecan anstelle von 1-Bromdodecan eingesetzt wurde, wobei 1,1'-(5-Ethyl-2-octadecyloxy-1,3-phenylen)-bis(N,N-dimethylmethanamin) als cremefarbener, wachsartiger Feststoff erhalten wurde (Ausbeute: 1,61 g; 66,0 % d.Th.).

### Stufe 3:

Die Reaktion erfolgte analog zu jener in Beispiel 13, wobei die Titelverbindung (15) als gelblicher, wachsartiger Feststoff erhalten wurde (Ausbeute: 1,00 g; 96,2 % d.Th.).

**¹H-NMR:** δ_{H} (300 MHz, CDCl₃) 7,51 (s, 2H, C2, C4), 4,40 (s, 4H, C8, C11), 3,72 (t, *J* = 6,8 Hz, 2H, C19), 3,08 (s, 12H, C13, C14, C16, C17), 2,65 (q, *J* = 7,7 Hz, 2H, C9), 1,88-1,77 (m, 2H, C20), 1,45-1,36 (m, 2H, C21), 1,26-1,18 (m, 33H, C10, C22-C35), 0,84 (t, *J* = 7,0 Hz, 3H, C36). **¹³C-NMR**: δ_{C} (75 MHz, CDCl₃) 156,2 (C6), 141,2 (C3), 136,3 (C1, C5), 124,6 (C2, C4), 70,2 (C8, C11), 58,1 (C13, C14, C16, C17), 32,0 (C20), 30,5 (C21), 29,9-29,6 (m) (C22-C31), 29,5 (C32), 29,4 (C33), 28,1 (C9), 26,2 (C34), 22,8 (C35), 15,3 (C10), 14,2 (C36). **HRMS:** (ESI⁺, m/z) ber. für C₃₂H₆₁NO₃ [M+H]⁺: 521,46822; gef.: 521,467246.

### Beispiel 16

Herstellung von 1,4-Bis(5-ethyl-3-methoxy-2-octyloxybenzyl)piperazin-1,4-dioxid (16)

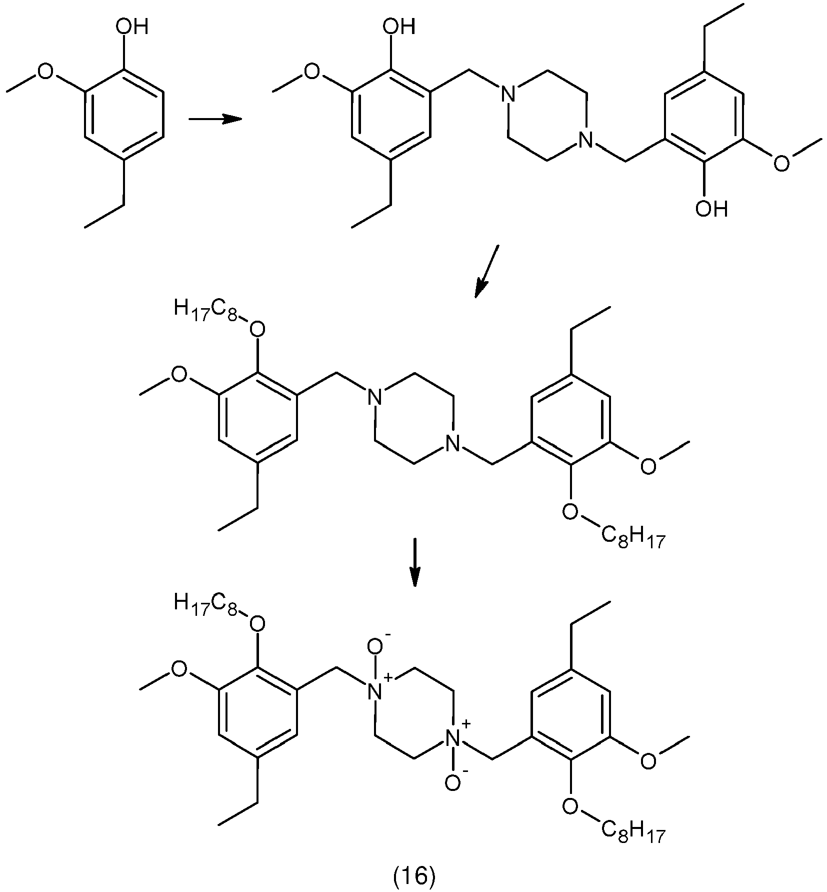

### Stufe 1:

Eine wässrige Lösung von 4-Ethylguajacol (3,04 g, 20 mmol) wurde binnen 15 min unter konstantem Rühren zu einer wässrigen Lösung (10 ml) von Piperazin (1,29 g, 15 mmol) in einem Eiswasserbad zugetropft. Dazu wurde eine 37-gew.-%ige wässrige Formaldehyd-Lösung (0,90 g, 30 mmol) in Aliquoten von jeweils 0,1 g alle 10 min zugesetzt und 3 h lang im Eiswasserbad und danach 9 h lang bei Raumtemperatur gerührt. Anschließend wurde der ausgefallene Feststoff abfiltriert (Glasfritte, Porosität 4), in 10 ml Petrolether aufgeschlämmt und mittels Ultraschall vermischt, danach abzentrifugiert und im Vakuumexsikkator vollständig getrocknet, wobei das dimere Zwischenprodukt, 6,6'-(Piperazin-1,4-dimethylen)-bis(4-ethyl-2-methoxyphenol), in Form von weißen Nadeln erhalten wurde (Ausbeute: 3,40 g; 82,1 % d.Th.).

### Stufe 2:

Die Reaktion erfolgte analog zu jener in Beispiel 1, Variante 2.2, außer dass 2,12 g (11 mmol) 1-Bromoctan und 40 ml 2-MeTHF als Lösungsmittel eingesetzt wurden und bei der Flash-Chromatographie die Elution mit einem Petrolether/Ethylacetat-Gradienten erfolgte, wobei 1,4-Bis(5-ethyl-3-methoxy-2-octyloxybenzyl)piperazin als weißes Pulver erhalten wurde (Ausbeute: 1,45 g; 45,4 % d.Th.).

### Stufe 3:

Die Reaktion erfolgte analog zu jener in Beispiel 1, außer dass nur 1 mmol 1,4-Bis(5-ethyl-3-methoxy-2-octyloxybenzyl)piperazin und Ameisensäuremethylester (15 ml) als zusätzliches Lösungsmittel eingesetzt wurden, wobei die Titelverbindung (16) als weißer, wachsartiger Feststoff erhalten wurde (Ausbeute: 0,21 g; 31,6 % d.Th.).

**¹H-NMR:** δ_{H} (300 MHz, CDCl₃) 6,94 (d, *J* = 2,0 Hz, 2H), 6,77 (d, *J* = 2,0 Hz, 2H), 4,45 (s, 4H), 4,28 (d, *J* = 8,3 Hz, 4H), 3,93 (t, *J* = 7,0 Hz, 4H), 3,82 (s, 6H), 3,02 (d, *J* = 8,1 Hz, 4H), 2,59 (q, *J* = 7,6 Hz, 4H), 1,74 (q, *J* = 7,0 Hz, 4H), 1,44-1,15 (m, 26H), 0,93-0,82 (m, 6H).**¹³C-NMR**: δ_{C} (75 MHz, CDCl₃) 152,4, 139,8, 125,2, 122,4, 114,1, 73,7, 58,6, 55,9, 32,0, 30,4, 29,5, 29,4, 28,6, 26,0, 22,8, 15,4, 14,3. **HRMS:** (ESI⁺, m/z) ber. für C₄₀H₆₇N₂O₆ [M+H]⁺: 671,49991; gef.: 671,498929.

### Beispiel 17

Herstellung von 1,4-Bis(2-dodecyloxy-5-ethyl-3-methoxybenzyl)piperazin-1,4-dioxid (17)

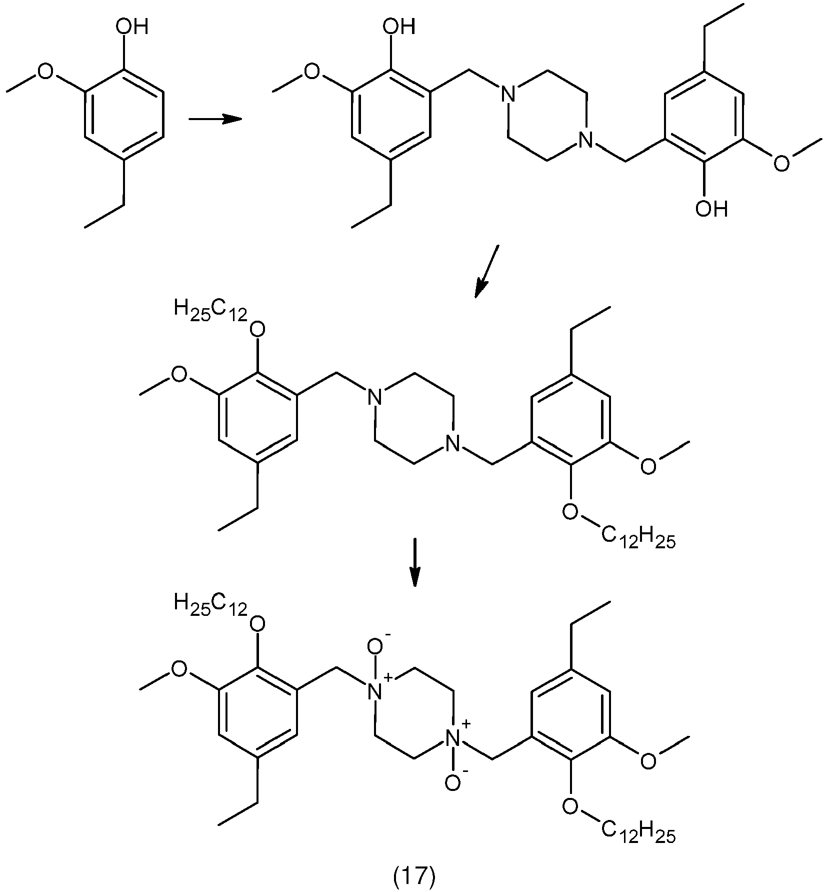

### Stufe 1:

Die Synthese und das Produkt waren identisch mit Beispiel 16.

### Stufe 2:

Die Reaktion erfolgte analog zu jener in Beispiel 16, außer dass 1-Bromdodecan anstelle von 1-Bromoctan eingesetzt wurde, wobei 1,4-Bis(2-dodecyloxy-5-ethyl-3-methoxybenzyl)piperazin als weißes Pulver erhalten wurde (Ausbeute: 1,61 g; 79,6 % d.Th.).

**¹H-NMR:** δ_{H} (600 MHz, CDCl₃) 6,77 (2 H, d, *J* 2,0), 6,63 (2 H, d, *J* 2,0), 3,89 (4 H, t, *J* 6,8), 3,82 (6 H, s), 3,52 (4 H, s), 2,66-2,32 (12 H, m), 1,75 (4 H, p, *J* 6,9), 1,48-1,40 (4 H, m), 1,37-1,23 (36 H, m), 1,22 (6 H, t, *J* 7,6), 0,88 (6 H, t, *J* 7,0). **¹³C-NMR**: δ_{C} (151 MHz, CDCl₃) 152,7, 145,2, 139,5, 131,8, 121,9, 110,9, 73,6, 56,8, 55,9, 53,4, 32,1, 30,5, 29,9-29,8 (m), 29,7, 29,5, 28,9, 26,3, 22,8, 15,8, 14,3. **Elementaranalyse:** Ber.: C, 76,75; H, 11,00; N, 3,73; gef.: C, 76,18; H, 10,51; N, 3,67; **HRMS:** (ESI⁺, m/z) ber. für C₄₈H₈₃N₂O₄ [M+H]⁺: 751,63474; gef.: 751,63595.

### Stufe 3:

Die Reaktion erfolgte analog zu jener in Beispiel 1, wobei jedoch aufgrund der schlechten Wasserlöslichkeit des 1,4-Bis(2-dodecyloxy-5-ethyl-3-methoxybenzyl)piperazins nur äußerst geringer Umsatz zu beobachten war. Die Optimierung dieser Oxidationsreaktion mit Ameisensäuremethylester als zusätzlichem Lösungsmittel ist derzeit Gegenstand der Forschung der Erfinder.

### Beispiel 18

Herstellung von N,N'-Bis(5-ethyl-3-methoxy-2-octyloxybenzyl)-N,N'-dimethylethan-1,2-diamin-di-N-oxid (18)

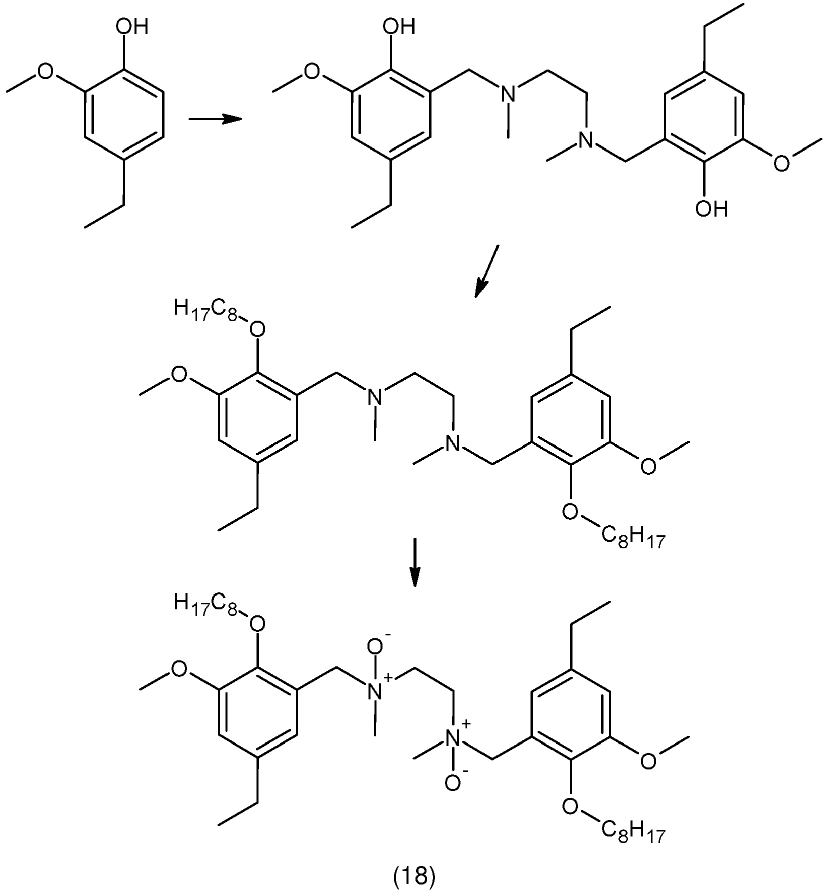

### Stufe 1:

Die Reaktion erfolgte analog zu jener in Beispiel 16, außer dass N,N'-Dimethylethylendiamin anstelle von Piperazin eingesetzt wurde, wobei 6,6'-(N,N'-Dimethylethan-1,2-diamin-N,N'-dimethylen)-bis(4-ethyl-2-methoxyphenol) als leicht gelbliches Pulver erhalten wurde (Ausbeute: 1,90 g; 91,4 % d.Th.).

### Stufe 2:

Die Reaktion erfolgte analog zu jener in Beispiel 16, wobei N,N'-Bis(5-ethyl-3-methoxy-2-octyloxybenzyl)-N,N'-dimethylethan-1,2-diamin als cremefarbenes Pulver erhalten wurde (Ausbeute: 0,91 g; 28,5 % d.Th.).

### Stufe 3:

Die Reaktion erfolgte analog zu jener in Beispiel 16 mit Ameisensäuremethylester (15 ml) als zusätzlichem Lösungsmittel, wobei die Titelverbindung (17) als weißer, wachsartiger Feststoff erhalten wurde. Da es allerdings im Verlauf der Oxidation zu Zersetzungsreaktionen des Diamins kam, fiel das gewünschte Produkt im Gemisch mit einer Reihe von Nebenprodukten an, die auch mittels Säulenchromatographie kaum zu trennen waren. Die Optimierung dieser Oxidationsreaktion ist derzeit Gegenstand der Forschung der Erfinder.

### Beispiel 19

Tests bezüglich der Eignung der isolierten Amin-N-oxid-Verbindungen der Formel (I) und (II) als Tenside

Als Parameter für die Tensideigenschaften der neuen Amin-N-oxid-Verbindungen wurde wie üblich die kritische Mizellenbildungskonzentration ("critical micelle concentration", CMC), d.h. die Konzentration, ab der sich Mizellen bilden können, mittels eines K100C Force Tensiometers von Krüss Scientific gemäß der Wilhelmy-Platten-Methode bei 25 °C und pH 3 bestimmt. Zum Vergleich wurde Dodecyldimethylamin-N-oxid ("Vergleich") unter den gleichen Bedingungen vermessen. Die Ergebnisse sind in der nachfolgenden Tabelle 1 angegeben, wobei niedrigere Werte eine stärkere Tensidwirkung der jeweiligen Substanz anzeigen.

**Tabelle 1**

| **Verbindung** | **CMC (mg/l)** | **Verbindung** | **CMC (mg/l)** |
|---|---|---|---|
| (1) | 1,4 x 10³ | (7) | 15 |
| (2) | 2,1 x 10² | (8) | 55 |
| (3) | 35 | (13) | 85 |
| (4) | 5,2 | (14) | 37 |
| (5) | 1,8 | (15) | 15 |
| (6) | 1,3 | V1 | 9,6 x 10² |

Man erkennt, dass die erfindungsgemäßen Beispiele mit Ausnahme von Beispiel 1 niedrigere ― und zwar mehrheitlich deutlich niedrigere ― CMC-Werte aufweisen als die in einer Vielzahl von im Handel erhältlichen Produkten eingesetzte Vergleichssubstanz. Allerdings belegt auch der CMC-Wert von 1,4 g/l für das Amin-N-oxid (1) aus Beispiel 1, das die geringste Anzahl an Kohlenstoffatomen in den Resten R¹ bis R⁵ enthält, durchaus dessen Eignung als Tensid.

Aufgrund der Analogien bzw. starken Ähnlichkeiten der Substitutionsmuster der übrigen, bisher noch nicht getesteten erfindungsgemäßen Verbindungen ist für den einschlägigen Fachmann zu erwarten, dass auch für diese mehrheitlich eine starke Tensidwirkung nachweisbar sein wird.

Die vorliegende Erfindung stellt somit eine Gruppe von neuen Amin-N-oxid-Verbindungen bereit, die mittels relativ einfacher Syntheseschritte auf wirtschaftliche und die Umwelt schonende Weise erhältlich sind und von denen sich die große Mehrzahl zur Verwendung als Tenside eignen.

## Patentansprüche

1. Amin-N-oxid-Verbindung der nachstehenden Formel (I) oder (II): 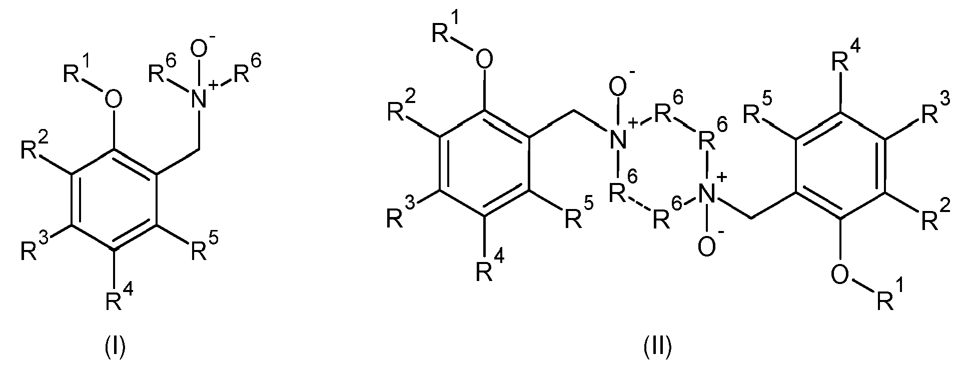 worin
R¹ aus linearen, verzweigten oder zyklischen Kohlenwasserstoff-Resten mit 4 bis 26 Kohlenstoffatomen, in denen gegebenenfalls zumindest ein Kohlenstoffatom durch ein Sauerstoff- oder Schwefelatom ersetzt ist, ausgewählt ist;
R², R³ und R⁵ jeweils unabhängig aus Wasserstoff, R¹-O-, R⁸ und in Formel (I) auch aus -CH₂-N⁺(O⁻)R⁶R⁶ ausgewählt sind, wobei R⁸ für einen linearen, verzweigten oder zyklischen Kohlenwasserstoff-Rest mit 1 bis 26 Kohlenstoffatomen, in dem gegebenenfalls zumindest ein Kohlenstoffatom durch ein Sauerstoff- oder Schwefelatom ersetzt ist, steht;
R⁴ aus Wasserstoff und R⁸ ausgewählt ist; und
die Reste R⁶ jeweils unabhängig aus gesättigten, linearen oder verzweigten Kohlenwasserstoff-Resten mit 1 bis 6 Kohlenstoffatomen, in denen gegebenenfalls zumindest ein Kohlenstoffatom durch ein Stickstoff-, Sauerstoff- oder Schwefelatom ersetzt ist, ausgewählt sind;
wobei gegebenenfalls zwei an dasselbe Stickstoffatom gebundene Reste R⁶ miteinander zu einem fünf- oder sechsgliedrigen stickstoffhältigen Ring verbunden sind oder
wobei gegebenenfalls ein Rest R⁶ oder beide Reste R⁶ einer Amin-N-oxid-Gruppierung -N⁺(O⁻)R⁶R⁶ mit einem oder beiden Resten R⁶ einer solchen Gruppierung eines anderen Moleküls der Formel (I) verbunden sind und unter Ausbildung einer Brücke mit der Struktur 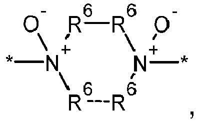 worin die gestrichelte Linie eine optionale Bindung zwischen den beiden Resten R⁶ und die Sternchen die Anbindungen der Brücke an die beiden aromatischen Ringe anzeigen, ein Dimer gemäß Formel (II) bilden.

2. Amin-N-oxid-Verbindung nach Anspruch 1, **dadurch gekennzeichnet, dass**
R¹ C₆-C₂₂-Alkyl ist; und/oder
R² aus C₁-C₂₂-Alkyl, C₁-C₂₂-Alkoxy und -CH₂-N⁺(O⁻)R⁶R⁶ ausgewählt ist; und/oder
R³ und R⁵ aus Wasserstoff und -CH₂-N⁺(O⁻)R⁶R⁶ ausgewählt sind; und/oder
R⁴ aus Wasserstoff, C₁-C₄-Alkyl und C₁-C₄-Alkoxy ausgewählt ist.

3. Amin-N-oxid-Verbindung nach Anspruch 2, **dadurch gekennzeichnet, dass**
R¹ C₈-C₁₈-Alkyl ist; und/oder
R² aus C₁-C₁₈-Alkoxy und -CH₂-N⁺(O⁻)R⁶R⁶ ausgewählt ist; und/oder
R⁴ und R⁵ aus Wasserstoff und C₁-C₄-Alkyl ausgewählt sind.

4. Amin-N-oxid-Verbindung nach Anspruch 2, **dadurch gekennzeichnet, dass**
R¹ C₈-C₁₈-Alkyl ist;
R² C₁-C₁₈-Alkoxy ist;
einer von R³ und R⁵ Wasserstoff ist und der andere -CH₂-N⁺(O⁻)R⁶R⁶ ist; und
R⁴ C₁-C₄-Alkyl ist.

5. Amin-N-oxid-Verbindung nach Anspruch 2, **dadurch gekennzeichnet, dass**
R¹ C₈-C₁₈-Alkyl ist;
R² C₁-C₁₈-Alkoxy ist;
R³ und R⁵ jeweils Wasserstoff sind; und
R⁴ C₁-C₄-Alkyl ist.

6. Amin-N-oxid-Verbindung nach Anspruch 5, **dadurch gekennzeichnet, dass**
R² Methoxy ist; und
R⁴ Ethyl oder Propyl ist.

7. Amin-N-oxid-Verbindung nach Anspruch 2, **dadurch gekennzeichnet, dass**
R¹ C₈-C₁₈-Alkyl ist;
R² -CH₂-N⁺(O⁻)R⁶R⁶ ist;
R³ und R⁵ jeweils Wasserstoff sind; und
R⁴ C₁-C₄-Alkyl ist.

8. Amin-N-oxid-Verbindung nach einem der Ansprüche 1 bis 7, **dadurch gekennzeichnet, dass**
die Reste R⁶ jeweils unabhängig aus Methyl, Ethyl und Dimethylaminoethyl ausgewählt sind; und/oder
zwei an dasselbe Stickstoffatom gebundene Reste R⁶ miteinander verbunden sind und zusammen mit dem Stickstoffatom eine der nachstehenden Gruppen bilden: 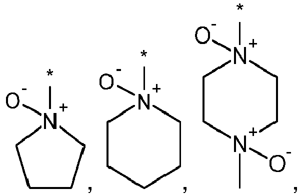 wobei das Sternchen jeweils die Anbindung an den aromatischen Ring anzeigt; und/oder
die Reste R⁶ jeweils Methyl sind und eine oder beide Methylgruppen einer Gruppierung -N⁺(O⁻)(CH₃)₂ mit einer oder beiden Methylgruppen einer solchen Gruppierung eines anderen Moleküls der Formel (I) verbunden sind und unter Ausbildung einer Brücke mit der Struktur 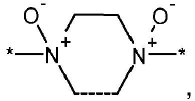 worin die gestrichelte Linie eine optionale Bindung zwischen den beiden Methylgruppen und die Sternchen die Anbindungen der Brücke an die beiden aromatischen Ringe anzeigen, ein Dimer der Amin-N-oxid-Verbindung gemäß Formel (II) bilden.

9. Amin-N-oxid-Verbindung nach einem der Ansprüche 1 bis 8, **dadurch gekennzeichnet, dass** sie aus den folgenden Verbindungen ausgewählt ist:
N,N-Dimethyl-1-(5-ethyl-3-methoxy-2-octyloxyphenyl)methanamin-N-oxid (1) 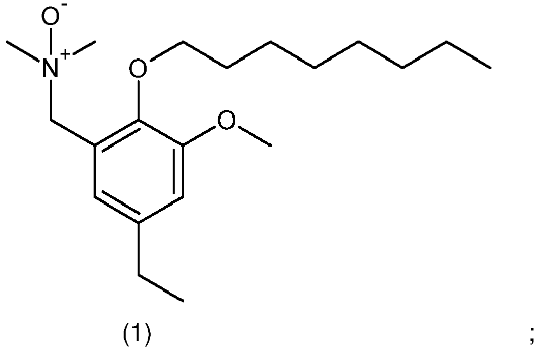
N,N-Dimethyl-1-(2-decyloxy-5-ethyl-3-methoxyphenyl)methanamin-N-oxid (2) 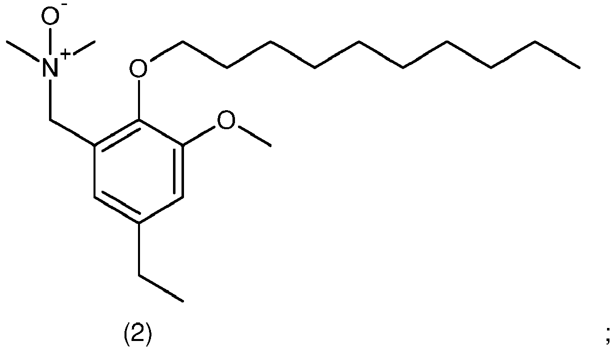
N,N-Dimethyl-1-(2-dodecyloxy-5-ethyl-3-methoxyphenyl)methanamin-N-oxid (3) 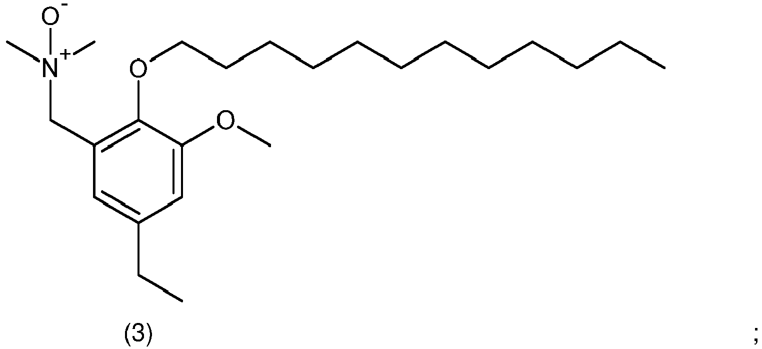
N,N-Dimethyl-1-(5-ethyl-3-methoxy-2-tetradecyloxyphenyl)methanamin-N-oxid (4) 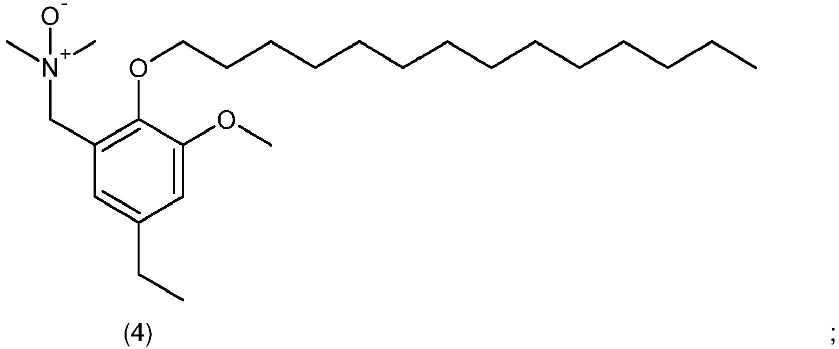
N,N-Dimethyl-1-(5-ethyl-2-hexadecyloxy-3-methoxyphenyl)methanamin-N-oxid (5) 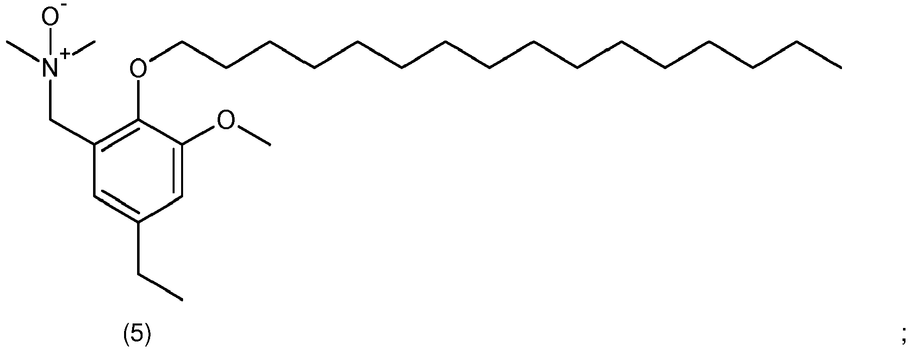
N,N-Dimethyl-1-(5-ethyl-3-methoxy-2-octadecyloxyphenyl)methanamin-N-oxid (6) 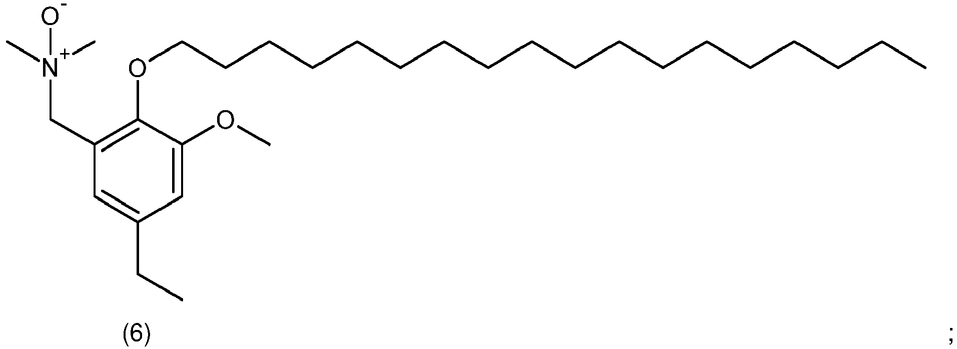
1-(2-Dodecyloxy-5-ethyl-3-methoxybenzyl)pyrrolidin-1-oxid (7) 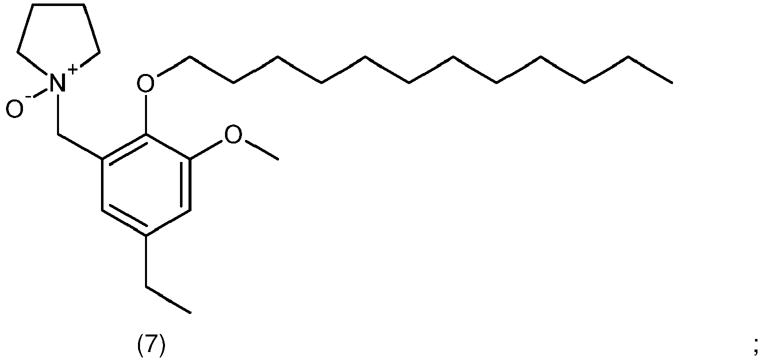
1-(2-Dodecyloxy-5-ethyl-3-methoxybenzyl)piperidin-1-oxid (8) 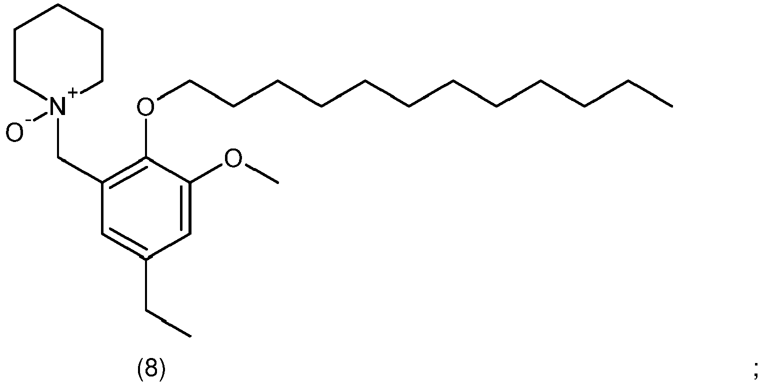
1-(2-Dodecyloxy-5-ethyl-3-methoxybenzyl)-4-methylpiperazin-1,4-dioxid (9) 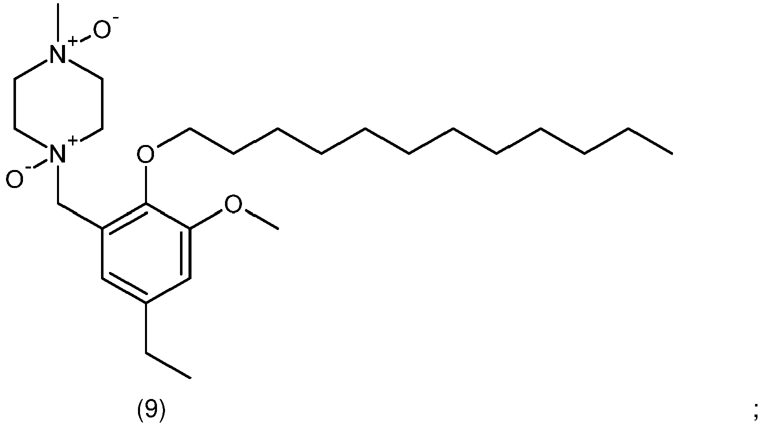
N,N-Dimethyl-N'-(2-dodecyloxy-5-ethyl-3-methoxybenzyl)-N'-methylethan-1,2-diamin-di-N-oxid (10) 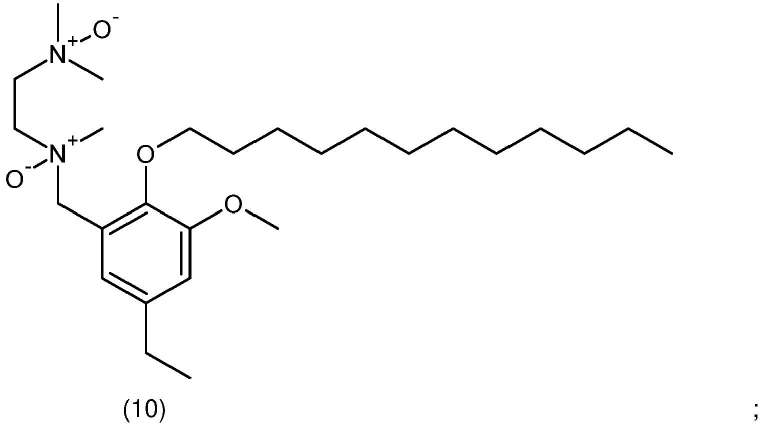
1,1'-(2,3-Dioctyloxy-5-ethyl-1,4-phenylen)-bis(N,N-dimethylmethanamin-N-oxid) und 1,1'-(2,3-Dioctyloxy-5-ethyl-1,6-phenylen)-bis(N,N-dimethylmethanamin-N-oxid) (11) 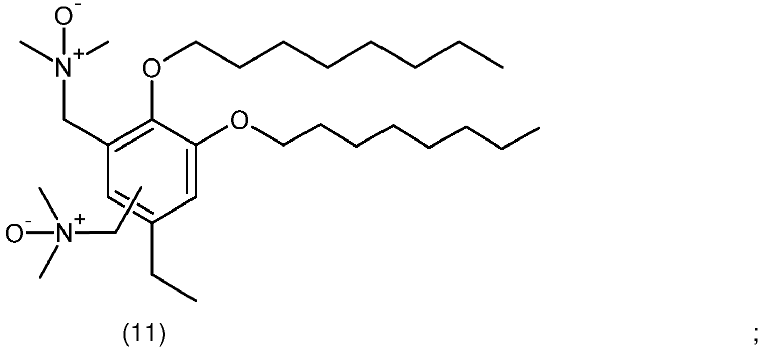
1,1'-(5-Ethyl-2-octyloxy-1,3-phenylen)-bis(N,N-dimethylmethanamin-N-oxid) (12) 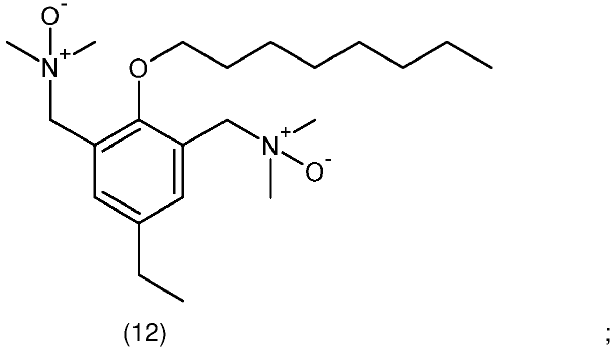
1,1'-(2-Dodecyloxy-5-ethyl-1,3-phenylen)-bis(N,N-dimethylmethanamin-N-oxid) (13) 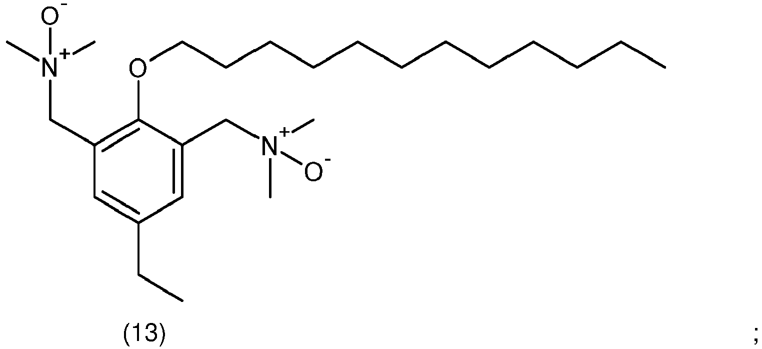
1,1'-(5-Ethyl-2-hexadecyloxy-1,3-phenylen)-bis(N,N-dimethylmethanamin-N-oxid) (14) 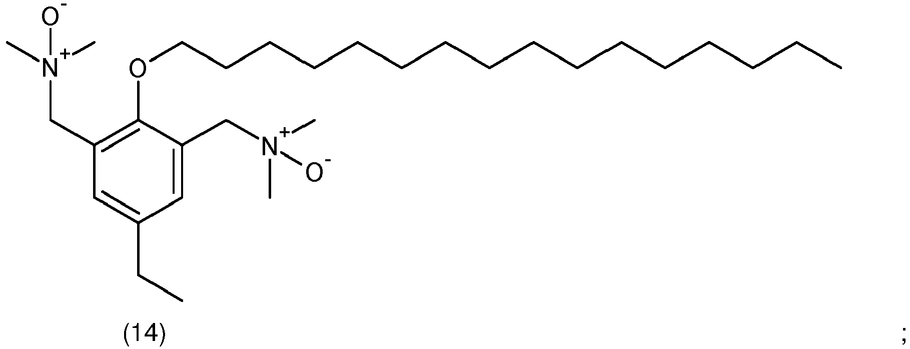
1,1'-(5-Ethyl-2-octadecyloxy-1,3-phenylen)-bis(N,N-dimethylmethanamin-N-oxid) (15) 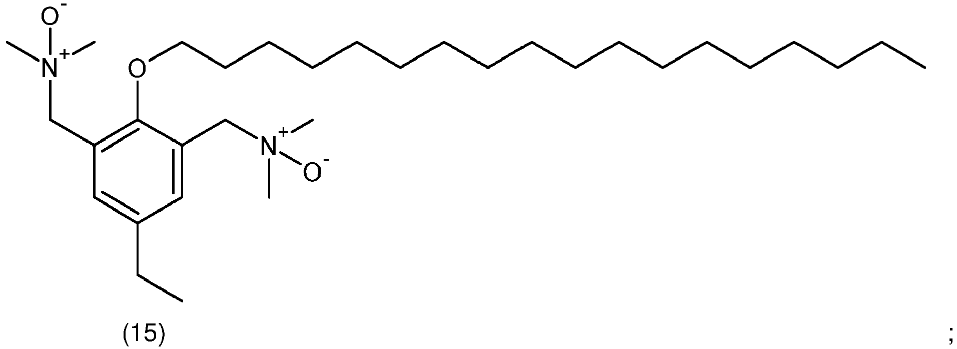
1,4-Bis(5-ethyl-3-methoxy-2-octyloxybenzyl)piperazin-1,4-dioxid (16) 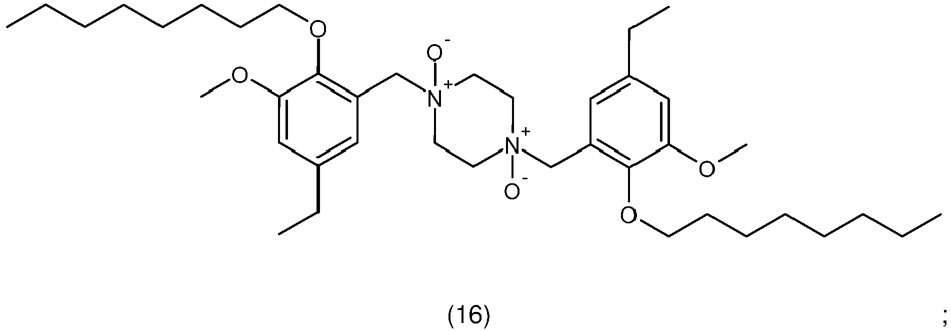
1,4-Bis(2-dodecyloxy-5-ethyl-3-methoxybenzyl)piperazin-1,4-dioxid (17) 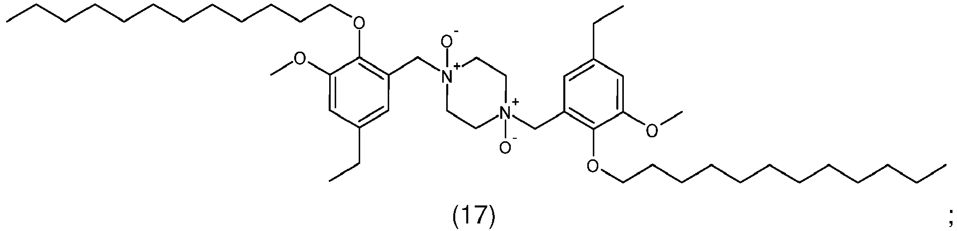 N,N'-Bis(5-ethyl-3-methoxy-2-octyloxybenzyl)-N,N'-dimethylethan-1,2-diamin-di-N-oxid (18)

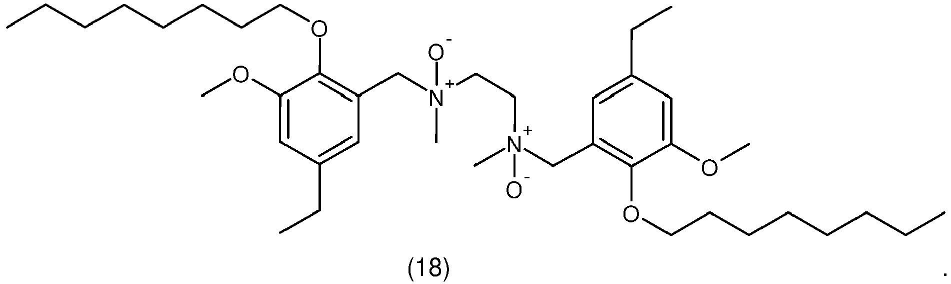

10. Verfahren zur Herstellung einer Amin-N-oxid-Verbindung nach einem der Ansprüche 1 bis 9, das die folgenden Schritte umfasst:
1) das Umsetzen eines Phenol-Derivats der nachstehenden Formel (III): 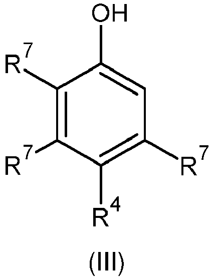 worin
R⁴ aus Wasserstoff und R⁸ ausgewählt ist, wobei R⁸ für einen linearen, verzweigten oder zyklischen Kohlenwasserstoff-Rest mit 1 bis 26 Kohlenstoffatomen, in dem gegebenenfalls zumindest ein Kohlenstoffatom durch ein Sauerstoff- oder Schwefelatom ersetzt ist, steht, und
die R⁷ jeweils unabhängig aus Wasserstoff, Hydroxy und R⁸ ausgewählt sind,
mit einem sekundären Amin HNR⁶R⁶, worin die Reste R⁶ jeweils unabhängig aus gesättigten, linearen oder verzweigten Kohlenwasserstoff-Resten mit 1 bis 6 Kohlenstoffatomen, in denen gegebenenfalls zumindest ein Kohlenstoffatom durch ein Stickstoff-, Sauerstoff- oder Schwefelatom ersetzt ist, ausgewählt sind,
wobei gegebenenfalls die beiden Reste R⁶ miteinander zu einem fünf- oder sechsgliedrigen stickstoffhältigen Ring verbunden sind oder
wobei gegebenenfalls jeweils ein Rest R⁶ oder jeweils beide Reste R⁶ zweier Moleküle des sekundären Amins unter Ausbildung eines Dimers des sekundären Amins mit der Struktur 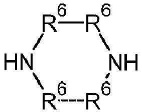 miteinander verbunden sind, worin die gestrichelte Linie eine optionale Bindung zwischen den beiden Resten R⁶ anzeigt,
mittels einer Aminoalkylierungsreaktion nach Betti/Mannich in Gegenwart von Formaldehyd in einem polaren Lösungsmittel, wodurch das Wasserstoffatom in ortho-Stellung zur phenolischen OH-Gruppe und gegebenenfalls ein weiteres substituierbares Wasserstoffatom R⁷ des Phenol-Derivats der Formel (II) durch eine Gruppierung -CH₂-NR⁶R⁶ ersetzt wird/werden und eine entsprechende Betti-Base der Formel (IV) oder (V) erhalten wird: 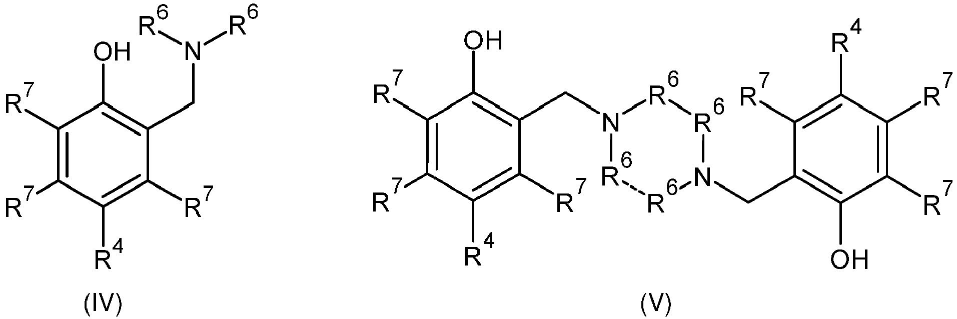
worin die R⁷ jeweils unabhängig aus Wasserstoff, Hydroxy, R⁸ und in Formel (IV) auch aus -CH₂-NR⁶R⁶ ausgewählt sind und die gestrichelte Linie eine optionale Bindung zwischen den beiden Resten R⁶ anzeigt;
2) das Umsetzen der (beiden) phenolischen OH-Gruppe(n) und gegebenenfalls weiterer freier OH-Gruppen R⁷ der jeweiligen Betti-Base der Formel (IV) oder (V) mit einer Verbindung der Formel R¹-X, worin R¹ aus linearen, verzweigten oder zyklischen Kohlenwasserstoff-Resten mit 4 bis 26 Kohlenstoffatomen, in denen gegebenenfalls zumindest ein Kohlenstoffatom durch ein Sauerstoff- oder Schwefelatom ersetzt ist, ausgewählt ist und X für eine aus Halogeniden und Sulfonaten ausgewählte Abgangsgruppe steht, mittels einer Veretherungsreaktion nach Williamson in Gegenwart einer Base in einem organischen Lösungsmittel oder ohne Lösungsmittel, wodurch ein entsprechender Ether der Formel (VI) oder (VII) erhalten wird: 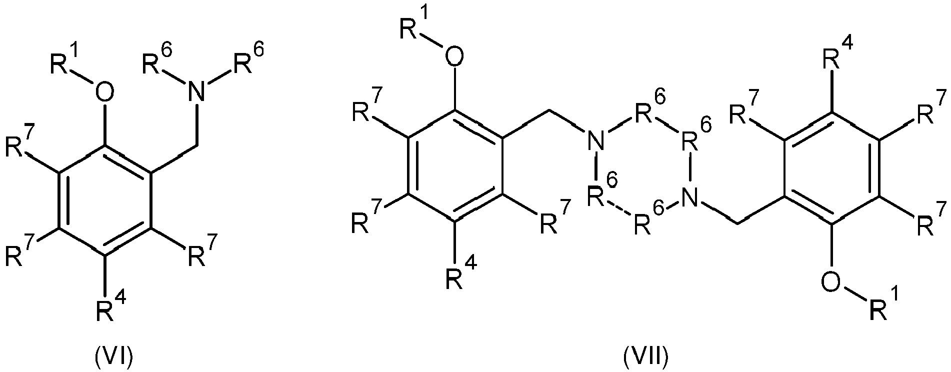 worin die R⁷ jeweils unabhängig aus Wasserstoff, R¹-O-, R⁸ und in Formel (VI) auch aus -CH₂-NR⁶R⁶ ausgewählt sind und die gestrichelte Linie eine optionale Bindung zwischen den beiden Resten R⁶ anzeigt;
3) das Oxidieren jeglicher Aminogruppen -NR⁶R⁶ des jeweiligen Ethers der Formel (VI) oder (VII) durch Umsetzung mit einem Oxidationsmittel in Wasser, einem organischen Lösungsmittel oder einem Gemisch davon, wodurch die Amin-N-oxid-Verbindung der Formel (I) oder (II) erhalten wird: 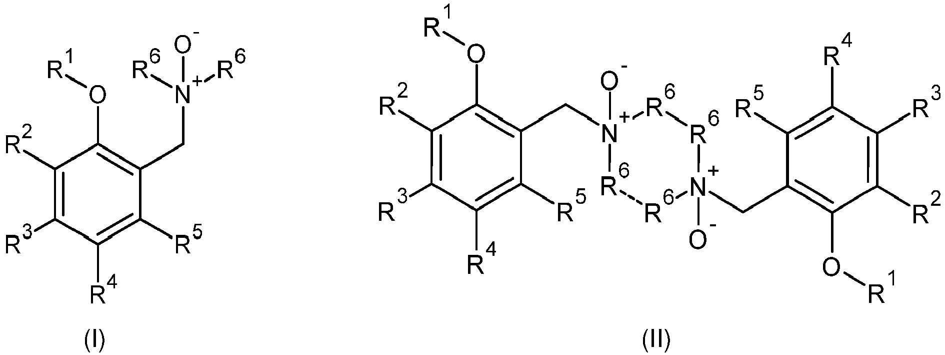 worin R², R³ und R⁵ jeweils unabhängig aus Wasserstoff, R¹-O-, R⁸ und in Formel (I) auch aus -CH₂-N⁺(O⁻)R⁶R⁶ ausgewählt sind und die gestrichelte Linie eine optionale Bindung zwischen den beiden Resten R⁶ anzeigt.

11. Verfahren nach Anspruch 10, **dadurch gekennzeichnet, dass** in Schritt 1) das Phenol-Derivat der Formel (III) mit jeweils 1,5 Äquivalenten des sekundären Amins und von Formaldehyd umgesetzt wird,
wobei die Reaktion gegebenenfalls in Wasser bei Raumtemperatur durchgeführt wird.

12. Verfahren nach Anspruch 10 oder 11, **dadurch gekennzeichnet, dass** in Schritt 2) jeweils
eine Fest-Flüssig-Phasentransferreaktion unter Verwendung einer festen Base und in Gegenwart eines Phasentransfer-Katalysators durchgeführt wird; und/oder
als Abgangsgruppe X das Chlorid oder Bromid eingesetzt wird; und/oder ein wasserfreies Lösungsmittel eingesetzt wird.

13. Verfahren nach Anspruch 12, **dadurch gekennzeichnet, dass** in Schritt 2)
als feste Base gepulverte KOH eingesetzt wird;
als Phasentransfer-Katalysator Tetra-n-butylammoniumbromid (TBAB) eingesetzt wird;
als Abgangsgruppe X das Bromid eingesetzt wird; und
als Lösungsmittel wasserfreies 2-Methyltetrahydrofuran eingesetzt wird.

14. Verfahren nach einem der Ansprüche 10 bis 13, **dadurch gekennzeichnet, dass** in Schritt 3)
eine wässrige Lösung von H₂O₂ als Oxidationsmittel eingesetzt wird, wobei gegebenenfalls Ameisensäuremethylester als zusätzliches Lösungsmittel zugesetzt wird; und/oder
der Ether der Formel (VI) oder (VII) mit 2,5 bis 3 Äquivalenten H₂O₂ umgesetzt wird.

15. Verwendung einer Amin-N-oxid-Verbindung der Formel (I) oder (II) nach einem der Ansprüche 1 bis 9 oder hergestellt nach einem der Ansprüche 10 bis 14, bei der die Gesamtanzahl der Kohlenstoffatome der Reste R¹ bis R⁵ zumindest 9 beträgt, als Tensid.
